(19) Europäisches Patentamt
European Patent Office
Office européen des brevets

(11) **EP 4 537 901 A2**

(12) **EUROPEAN PATENT APPLICATION**

(43) Date of publication:
**16.04.2025 Bulletin 2025/16**

(21) Application number: **24213767.7**

(22) Date of filing: **26.07.2019**

(51) International Patent Classification (IPC):
***A61P 13/12*** *(2006.01)*

(52) Cooperative Patent Classification (CPC):
**A61K 31/44; A61K 31/422; A61K 31/4245; A61K 31/506; A61K 45/06; A61P 13/12**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.07.2018 EP 18185871**

(62) Document number(s) of the earlier application(s) in accordance with Art. 76 EPC:
**19749657.3 / 3 826 679**

(27) Previously filed application:
**26.07.2019 EP PCT/EP2019/070251**

(71) Applicant: **F. Hoffmann-La Roche AG**
**4070 Basel (CH)**

(72) Inventor: **FORNONI, Alessia**
**Miami, 33143 (US)**

(74) Representative: **Klostermeyer-Rauber, Dörte**
**F. Hoffmann-La Roche AG**
**Patent Department**
**Grenzacherstrasse 124**
**4070 Basel (CH)**

Remarks:
This application was filed on 19-11-2024 as a divisional application to the application mentioned under INID code 62.

# (54) COMPOUNDS FOR USE IN TREATING KIDNEY DISORDERS

(57) The present disclosure relates to ABCA1 inducer compounds for use in treating kidney disorders, and in particular, chronic kidney diseases, glomerular diseases or proteinuric kidney diseases such as Alport syndrome, focal segmental glomerulosclerosis, and diabetic kidney disease.

EP 4 537 901 A2

## Description

### TECHNICAL FIELD

The present disclosure relates to ABCA1 inducer compounds for use in treating kidney disorders, and in particular, chronic kidney diseases, glomerular disease or proteinuric kidney diseases such as Alport syndrome, focal segmental glomerulosclerosis, and diabetic kidney disease.

### BACKGROUND

Chronic Kidney disease (CKD) leading to end stage renal disease (ESRD) requiring dialysis or kidney transplantation is a growing epidemic that affects millions of patients worldwide (Bello AK et al, JAMA, 317: 1864, 2017). While diabetes remains the most common cause of ESRD worldwide, other causes such as hypertension, cystic kidney diseases and glomerulonephritis contribute to a large portion of prevalent ESRD (USRDS database). Many of these disorders can present with proteinuria that range from mild proteinuria to severe nephrotic range proteinuria, with severe proteinuria representing a major risk factor to progression to ESRD. While renal replacement strategies improve patient mortality, current therapeutic strategies slow but not halt the progression of CKD. Several intervention studies have failed to demonstrate effectiveness. This is largely due to the fact that many of the interventions being tested so far target late stage of kidney diseases rather than early pathogenetic processes.

Currently, present treatment strategies consist in the use of angiotensin-converting enzyme (ACE) inhibitors and angiotensin-receptor blockers (ARBs) which help to reduce proteinuria and slow down the progression of glomerulosclerosis. So far, patients with proteinuric kidney disease have been treated with ACE inhibitors which include, but are not limited to: Benazepril, Cilazapril, Enalapril (Vasotec), Fosinopril (Monopril), Lisinopril (Zestril, Prinivil), Perinopril, Ramipril, Quinapril (Accupril), Trandolapril and ARBs include Candesartan (Atacand), Epresartan, Irbesartan, Losartan (Cozaar), Telmisartan, Valsartan. The only approved drug for the treatment of proteinuria in FSGS is ACTHar. Many off label use of other immunosuppressant have also been implemented in several proteinuric kidney diseases such as FSGS. These include prednisone (or steroids in general), rituximab, calcineurin inhibitors (cyclosporine and tacrolimus), rapamycin, abatacept, mychophenolate mophetyl. Other strategies such as coenzyme Q10, Fish Oil, Vitamin D derivatives, gluten free diet, allopurinol, spironolactone, LDL apheresis, plasmapheresis are being utilized. However, many of these treatments are based on case series, are not supported by randomized studies, are often accompanied by severe side effects, and are not designed to target specific pathogenetic mechanisms. Furthermore, response to any of these treatments is unpredictable. Therefore, currently used drugs fail to provide a safe and effective treatment. More specifically, there is long-time felt unfulfilled need for novel pharmaceutical agents, for the prevention or the treatment of patients suffering from glomerular disorders and more broadly chronic kidney diseases.

### SUMMARY

The inventors further investigated new therapeutic strategies for the treatment of patients with chronic kidney diseases, in particular primary glomerular diseases such as Alport Syndrome and FSGS and secondary glomerular diseases such as diabetic kidney disease (DKD). They have shown that some pyridine carboxamide compounds have highly promising effects in the treatment of such kidney diseases.

Pyridine carboxamides have been described as small molecule inducers of the ATP-binding cassette transporter A1 protein (ABCA1 inducers). Such pyridine carboxamides are for example described in International Patent Applications Publ. Nos. WO 2011/029827, WO 2012/032018, WO 2013/037703 and WO 2014180741.

Consequently, the present disclosure relates to a compound for use in treating kidney diseases, wherein said compound is an ABCA1 inducer compound.

In a specific embodiment, such ABCA1 inducer compound is represented by the formula I below:

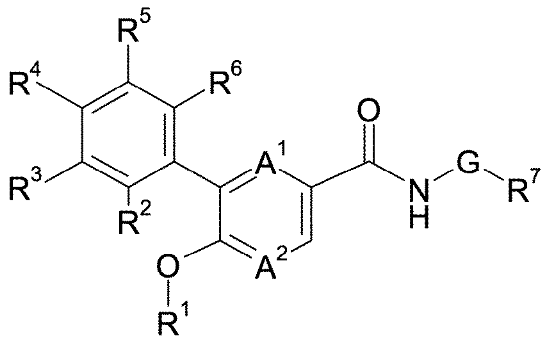

I

wherein

one of $A^1$ and $A^2$ is N and the other one of $A^1$ and $A^2$ is CH;
$R^1$ is selected from the group consisting of $C_{1-7}$-alkyl, $C_{3-7}$-cycloalkyl, $C_{3-7}$-cycloalkyl-$C_{1-7}$-alkyl, $C_{1-7}$-alkoxy-$C_{1-7}$-alkyl, halogen-$C_{1-7}$-alkyl, heterocyclyl-$C_{1-7}$-alkyl wherein the heterocyclyl group is unsubstituted or substituted by oxo, and heteroaryl-$C_{1-7}$-alkyl wherein the heteroaryl group is unsubstituted or mono-or di-substituted by lower alkyl;
$R^2$ and $R^6$ independently from each other are hydrogen or halogen;
$R^3$ and $R^5$ independently from each other are selected from the group consisting of hydrogen, $C_{1-7}$-alkyl, $C_{1-7}$-alkoxy, halogen, halogen-$C_{1-7}$-alkyl, halogen-$C_{1-7}$-alkoxy and cyano;
$R^4$ is selected from the group consisting of hydrogen, $C_{1-7}$-alkyl, $C_{1-7}$-alkoxy, halogen, halogen-$C_{1-7}$-alkyl, halogen-$C_{1-7}$-alkoxy, amino and cyano;
$R^7$ is selected from the group consisting of $C_{1-7}$-alkyl, $C_{3-7}$-cycloalkyl, said cycloalkyl being unsubstituted or substituted by hydroxy, heterocyclyl, said heterocyclyl having 3 to 7 ring atoms, comprising one, two or three heteroatoms selected from N, O and S and being unsubstituted or substituted by hydroxy or oxo, phenyl, wherein phenyl is unsubstituted or substituted by one or two groups selected from the group consisting of $C_{1-7}$-alkyl, hydroxy, $C_{1-7}$-alkoxy, cyano, halogen and halogen-$C_{1-7}$-alkyl, and heteroaryl, wherein heteroaryl is unsubstituted or substituted by one or two groups selected from the group consisting of $C_{1-7}$-alkyl, hydroxy, $C_{1-7}$-alkoxy, cyano, halogen and halogen-$C_{1-7}$-alkyl, and
G is selected from the group consisting of $-(CH2)_m-$, wherein m is selected from 0 or 1, and $-NR^{8}-$, wherein $R^8$ is hydrogen or $C_{1-7}$-alkyl,

and pharmaceutically acceptable salts thereof.

In specific embodiments, G is a bond and $R^7$ is $C_{3-7}$-cycloalkyl, said cycloalkyl being unsubstituted or substituted by hydroxy.

In specific embodiments that may be combined with the previous embodiments, $R^7$ is cyclohexyl substituted by hydroxy.

In specific embodiments that may be combined with the previous embodiments, $R^2$ and $R^6$ are each a hydrogen, $R^4$ is halogen, and one of $R^3$ and $R^5$ is halogen and the other one of $R^3$ and $R^5$ is hydrogen.

In specific embodiments that may be combined with the previous embodiments, $R^1$ is halogen-$C_{1-7}$-alkyl. Typically, $R^1$ may be selected from $-CF_3$, $-CHF_2$, $-CH_2Cl$, $-CH_2CF_3$, $-CH(CF_3)_2$, $-CF_2-CF_3$.

In a preferred embodiment, an ABCA1 inducer compound for use as described herein is 6-(3,4-dichlorophenyl)-N-[(1R,2R)-2-hydroxycyclohexyl]-5-(2,2,2-trifluoroethoxy)pyridine-2-carboxamide.

In another preferred embodiment, an ABCA1 inducer compound for use as described herein is 5-(3,4-dichlorophenyl)-N-((1R,2R)-2-hydroxy-cyclohexyl)-6-(2,2,2-trifluoro-ethoxy)-nicotinamide.

In specific embodiments that may be combined with the previous embodiments, the ABCA1 inducer compounds are useful in the treatment of chronic kidney diseases, primary and secondary glomerular diseases or proteinuric diseases. Typically, such ABCA1 inducer compound may be used in the treatment of Alport syndrome, focal segmental glomerulosclerosis, or diabetic kidney disease.

In specific embodiments that may be combined with the previous embodiments, the ABCA1 inducer compounds for use as described herein are formulated for oral administration.

In another specific embodiment, the ABCA1 inducer compounds for use as described herein are formulated for topical, intranasal, intraocular, intravenous, intramuscular, subcutaneous, intravitreal, intrathecal or transdermal administration.

The disclosure also relates to a method for treating kidney diseases in a subject in need thereof, comprising administering a therapeutically effective amount of an ABCA1 inducer as defined above.

In specific embodiments of such method, said ABCA1 inducer may be administered simultaneously, separately

or sequentially, in combination with a therapeutically effective amount of another agent, for example an angiotensin-converting enzyme inhibitor or an angiotensin-receptor blocker.

## DETAILED DESCRIPTION OF THE INVENTION

The inventors have shown that ABCA1 inducer compounds have highly promising effects in the treatment of kidney disorders, and in particular glomerular diseases, such as Alport syndrome or focal segmental glomerulosclerosis, or other chronic kidney diseases, such as diabetic kidney diseases. The compounds not only decrease proteinuria in these disorders but improve kidney function and prevent the development of end stage renal disease.

### *ABCA1 inducer compounds for use according to the present disclosure*

As used herein, the term "ABCA1 inducer compounds" refers to compounds capable of indirectly or directly inducing the expression level or activity of the ATP-binding cassette transporter protein (ABCA1). The transporter ABCA1 is known as a major regulator of cellular cholesterol and phospholipid homeostasis. In particular, ABCA1 mediates the efflux of cholesterol and phospholipids to lipid-poor apolipoproteins (apo-A1 and apoE) which then form nascent high-density lipoproteins (HDL). In vitro assays for determining upregulation of ABCA1 protein in cells have been described for example in WO2012/031817. Those in vitro assays include but are not restricted to cholesterol efflux assay or fluorescent apo-A1 binding assay as described in WO2012/031817.

Pyridine carboxamides have been described as small molecule inducers of the ATP-binding cassette transporter A1 protein (ABCA1 inducers). Such pyridine carboxamides are for example described in International Patent Applications Publ. Nos. WO 2011/029827, WO 2012/032018, WO 2013/037703 and WO 2014/180741.

In a preferred embodiment, the disclosure relates to the use of ABCA1 inducer compounds of the formula I

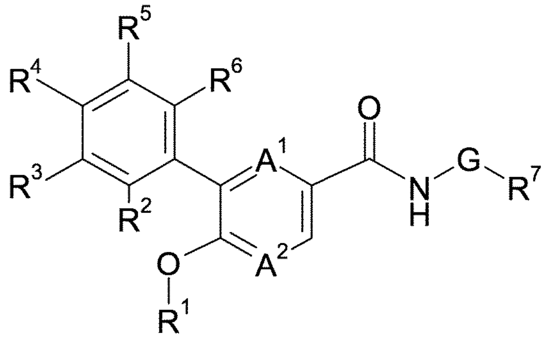

**I**

wherein one of $A^1$ and $A^2$ is N and the other one of $A^1$ and $A^2$ is CH;

$R^1$ is selected from the group consisting of $C_{1-7}$-alkyl, $C_{3-7}$-cycloalkyl, $C_{3-7}$-cycloalkyl-$C_{1-7}$-alkyl,$C_{1-7}$-alkoxy-$C_{1-7}$-alkyl, halogen-$C_{1-7}$-alkyl, heterocyclyl-$C_{1-7}$-alkyl wherein the heterocyclyl group is unsubstituted or substituted by oxo, and heteroaryl-$C_{1-7}$-alkyl wherein the heteroaryl group is unsubstituted or mono- or di-substituted by lower alkyl;
$R^2$ and $R^6$ independently from each other are hydrogen or halogen;
$R^3$ and $R^5$ independently from each other are selected from the group consisting of hydrogen, $C_{1-7}$-alkyl, $C_{1-7}$-alkoxy, halogen, halogen-$C_{1-7}$-alkyl, halogen-$C_{1-7}$-alkoxy and cyano;
$R^4$ is selected from the group consisting of hydrogen, $C_{1-7}$-alkyl, $C_{1-7}$-alkoxy, halogen, halogen-$C_{1-7}$-alkyl, halogen-$C_{1-7}$-alkoxy, amino and cyano;
$R^7$ is selected from the group consisting of $C_{1-7}$-alkyl, $C_{3-7}$-cycloalkyl, said cycloalkyl being unsubstituted or substituted by hydroxy,heterocyclyl, said heterocyclyl having 3 to 7 ring atoms, comprising one, two or three heteroatoms selected from N, O and S and being unsubstituted or substituted by hydroxy or oxo, phenyl, wherein phenyl is unsubstituted or substituted by one or two groups selected from the group consisting of $C_{1-7}$-alkyl, hydroxy, $C_{1-7}$-alkoxy, cyano, halogen and halogen-$C_{1-7}$-alkyl, and heteroaryl, wherein heteroaryl is unsubstituted or substituted by one or two groups selected from the group consisting of $C_{1-7}$-alkyl, hydroxy, $C_{1-7}$-alkoxy, cyano, halogen and halogen-$C_{1-7}$-alkyl, and
G is selected from the group consisting of-$(CH_2)_m$-, wherein m is selected from 0 or 1, and -$NR^8$-, wherein $R^8$ is hydrogen or $C_{1-7}$-alkyl, pharmaceutically acceptable salts thereof.

The term "lower alkyl" or "$C_{1-7}$-alkyl", alone or in combination, signifies a straight-chain or branched-chain alkyl group with 1 to 7 carbon atoms, in particular a straight or branched-chain alkyl group with 1 to 6 carbon atoms and more

particularly a straight or branched-chain alkyl group with 1 to 4 carbon atoms. Examples of straight-chain and branched $C_{1-7}$ alkyl groups are methyl, ethyl, propyl, isopropyl, butyl, isobutyl, tert-butyl, the isomeric pentyls, the isomeric hexyls and the isomeric heptyls, in particular methyl, ethyl, propyl, isopropyl and tert-butyl.

The term "lower alkoxy" or "$C_{1-7}$-alkoxy" refers to the group R'-O-, wherein R' is lower alkyl and the term "lower alkyl" has the previously given significance. Examples of lower alkoxy groups are methoxy, ethoxy, n-propoxy, isopropoxy, n-butoxy, isobutoxy, sec.-butoxy and tert.-butoxy, in particular methoxy.

The term "lower alkoxyalkyl" or "$C_{1-7}$-alkoxy-$C_{i-7}$-alkyl" refers to a lower alkyl group as defined above which is mono- or multiply substituted with a lower alkoxy group as defined above. Examples of lower alkoxyalkyl groups are e.g. $-CH_2-O-CH_3$, $-CH_2-CH_2-O-CH_3$, $-CH_2-O-CH_2-CH_3$ and the groups specifically exemplified herein. More particularly, lower alkoxyalkyl is methoxyethyl.

The term hydroxy means the group -OH.

The term "cycloalkyl" or "$C_{3-7}$-cycloalkyl" denotes a saturated carbocyclic group containing from 3 to 7 carbon atoms, such as cyclopropyl, cyclobutyl, cyclopentyl, cyclohexyl or cycloheptyl.

The term "lower cycloalkylalkyl" or "$C_{3-7}$-cycloalkyl-$C_{1-7}$-alkyl" refers to lower alkyl groups as defined above wherein at least one of the hydrogen atoms of the lower alkyl group is replaced by a cycloalkyl group as defined above. Among the lower cycloalkylalkyl groups of particular interest resides cyclopropylmethyl.

The term "halogen" refers to fluoro, chloro, bromo and iodo, with fluoro, chloro and bromo being of particular interest. More particularly, halogen refers to fluoro and chloro.

The term "lower halogenalkyl" or "halogen-$C_{1-7}$-alkyl" refers to lower alkyl groups which are mono- or multiply substituted with halogen, particularly with fluoro or chloro, most particularly with fluoro. Examples of lower halogenalkyl groups are e.g. - $CF_3$, $-CHF_2$, $-CH_2Cl$, $-CH_2CF_3$, $-CH(CF_3)_2$, $-CF_2-CF_3$, $-CH_2-CH_2-CF_3$, $-CH(CH_3)-CF_3$ and the groups specifically exemplified herein. Of particular interest are the groups trifluoromethyl ($-CF_3$) and 2,2,2-trifluoroethyl ($-CH_2CF_3$).

The term "lower halogenalkoxy" or "halogen-$C_{1-7}$-alkoxy" refers to lower alkoxy groups as defined above wherein at least one of the hydrogen atoms of the lower alkoxy group is replaced by a halogen atom, particularly fluoro or chloro, most particularly fluoro. Among the lower halogenalkoxy groups of particular interest are trifluoromethoxy, difluoromethoxy, fluormethoxy and chloromethoxy, more particularly trifluoromethoxy.

The term amino means the group $-NH_2$.

The term "cyano" means the group -CN.

The term "azido" means the group $-N_3$.

The term "heteroaryl" refers to an aromatic 5- or 6-membered ring which can comprise one, two or three atoms selected from N, O and S. Examples of heteroaryl groups are e.g. furanyl, pyridyl, pyrazinyl, pyrimidinyl, pyridazinyl, thienyl, isoxazolyl, thiazolyl, isothiazolyl, thiadiazolyl, oxazolyl, imidazolyl, pyrazolyl, triazolyl, oxadiazolyl, oxatriazolyl, tetrazolyl, pentazolyl, or pyrrolyl. The term "heteroaryl" also includes bicyclic groups comprising two 5- or 6-membered rings, in which one or both rings are aromatic and can contain one, two or three atoms selected from nitrogen, oxygen or sulphur, such as quinolinyl, isoquinolinyl, cinnolinyl, pyrazolo[1,5-a]pyridyl, imidazo[1,2-a]pyridyl, quinoxalinyl, benzothiazolyl, benzotriazolyl, indolyl, indazolyl, and 3,4-dihydro-2H-pyrido[3,2-b][1,4]oxazinyl. Heteroaryl groups of particular interest are of isoxazolyl, pyrazolyl, oxadiazolyl, thiazolyl, pyridyl, pyridazinyl, pyrimidinyl and pyrazinyl. More particularly, heteroaryl is pyridyl or pyridazinyl.

The term "lower heteroarylalkyl" or "heteroaryl-$C_{1-7}$-alkyl" refers to lower alkyl groups as defined above wherein at least one of the hydrogen atoms of the lower alkyl group is replaced by a heteroaryl group as defined above.

The term "heterocyclyl" refers to a saturated or partly unsaturated 3-, 4-, 5-, 6- or 7-membered ring which can comprise one, two or three heteroatoms selected from N, O and S. Examples of heterocyclyl rings include piperidinyl, piperazinyl, azetidinyl, azepinyl, pyrrolidinyl, pyrazolidinyl, imidazolinyl, imidazolidinyl, oxazolidinyl, isoxazolidinyl, morpholinyl, thiazolidinyl, isothiazolidinyl, oxiranyl, thiadiazolylidinyl, oxetanyl, dioxolanyl, dihydrofuranyl, tetrahydrofuranyl, dihydropyranyl, tetrahydropyranyl, and thiomorpholinyl. Of particular interest are piperidinyl and tetrahydropyranyl.

The term "lower heterocyclylalkyl" or "heterocyclyl-$C_{1-7}$-alkyl" refers to lower alkyl groups as defined above wherein at least one of the hydrogen atoms of the lower alkyl group is replaced by a heterocyclyl group as defined above.

The term "oxo" means that a C-atom of the heterocyclyl or heteroaryl ring may be substituted by =O, thus meaning that the heterocyclyl or heteroaryl ring may contain one or more carbonyl (-CO-) groups.

In specific embodiments for use of the compounds of Formula (I), G is a bond and $R^7$ is $C_{3-7}$-cycloalkyl, said cycloalkyl being unsubstituted or substituted by hydroxy. For example, $R^7$ is cyclohexyl that may or not be substituted by hydroxy.

In specific embodiments, $R^2$ and $R^6$ are each a hydrogen.

In specific embodiments, $R^4$ is halogen, for example chloro or fluoro, and one of $R^3$ and $R^5$ is halogen, for eample chloro or fluoro, and the other one of $R^3$ and $R^5$ is hydrogen.

In specific embodiments, $R_1$ is halogen-$C_{1-7}$-alkyl, typically, $R_1$ is selected from $-CF_3$, - $CHF_2$, $-CH_2Cl$, $-CH_2CF_3$, $-CH(CF_3)_2$, $-CF_2-CF_3$.

Examples of specific compounds are the following compounds:

| | |
|---|---|
| ***G*** | 5-(3,4-dichloro-phenyl)-N-((1*R*,2*R*)-2-hydroxy-cyclohexyl)-6-(2,2,2-trifluoro-ethoxy)-nicotinamide<br>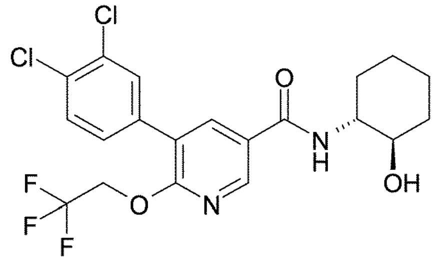 |
| ***A*** | 6-(3,4-dichlorophenyl)-N-[(1*R*,2*R*)-2-hydroxycyclohexyl]-5-(2,2,2-trifluoroethoxy)pyridine-2-carboxamide<br>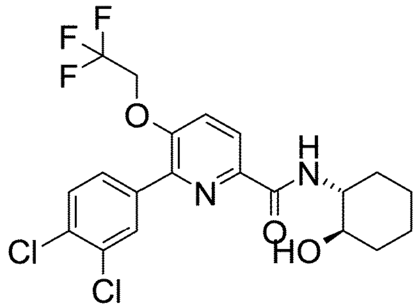 |
| ***H*** | *N*'-(6-chloropyridazin-3-yl)-5-(4-cyanophenyl)-*N*'-methyl-6-(2,2,2-trifluoroethoxy)pyridine-3-carbohydrazide<br>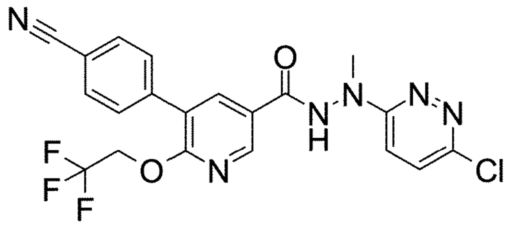 |
| ***J*** | 6-(4-chloro-phenyl)-5-(2,2,2-trifluoro-ethoxy)-pyridine-2-carboxylic acid (3-isopropyl-isoxazol-5-ylmethyl)-amide<br>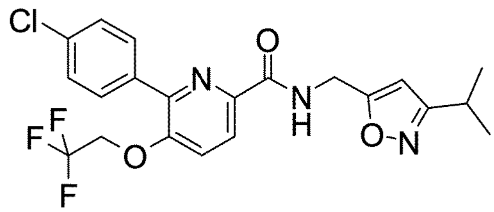 |

In specific embodiments, a compound for use in treating kidney diseases as described herein is selected from the group consisting of 5-(3,4-dichloro-phenyl)-N-((1*R*,2*R*)-2-hydroxy-cyclohexyl)-6-(2,2,2-trifluoro-ethoxy)-nicotinamide and 6-(3,4-dichlorophenyl)-N-[(1*R*,2*R*)-2-hydroxycyclohexyl]-5-(2,2,2-trifluoroethoxy)pyridine-2-carboxamide.

The disclosure also encompasses the compound of formula (I), tautomers, enantiomers, diastereomers, racemates or mixtures thereof, or hydrates, solvates, pharmaceutically acceptable salts for use in kidney diseases.

The term "pharmaceutically acceptable salts" refers to those salts which retain the biological effectiveness and properties of the free bases or free acids, and which do not possess any own properties that are undesirable. The salts are formed with inorganic acids such as hydrochloric acid, hydrobromic acid, sulfuric acid, nitric acid, phosphoric acid and the like, particularly hydrochloric acid, and organic acids such as formic acid, acetic acid, propionic acid, glycolic acid, pyruvic acid, oxalic acid, maleic acid, malonic acid, salicylic acid, succinic acid, fumaric acid, tartaric acid, citric acid, benzoic acid, cinnamic acid, mandelic acid, methanesulfonic acid, ethanesulfonic acid, *p*-toluenesulfonic acid, salicylic acid, *N*-acetylcystein and the like. Thus, in specific embodiments, "pharmaceutically acceptable salts" include the acetate, bromide, chloride, formate, fumarate, maleate, mesylate, nitrate, oxalate, phosphate, sulfate, tartrate and tosylate salt of compounds of formula I. In addition, pharmaceutically acceptable salts may be prepared from addition of an inorganic base

or an organic base to the free acid. Salts derived from an inorganic base include, but are not limited to, the sodium, potassium, lithium, ammonium, calcium, magnesium salts and the like. Salts derived from organic bases include, but are not limited to salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, diethylamine, lysine, arginine, *N*-ethylpiperidine, piperidine, piperazine and the like. The compound of formula I can also be present in the form of zwitterions or in the form of hydrates. Particularly pharmaceutically acceptable salts of compounds of formula I may be hydrochloride salts.

Examples of synthesis methods of the compounds of formula I are described in WO2011/029827, WO 2012/032018, WO 2013/37703 and WO2014/180741. An exemplary method for the preparation of compound ***G*** is described in WO2011/029827 and an exemplary method for the preparation of compound ***A*** is disclosed in WO2014/180741.

***Methods of use of the compounds of formula I***

The compounds of formula I, typically, compounds ***A*** and ***G*** as described in the examples, or their pharmaceutically acceptable salts, have been shown to be useful in the treatment of kidney diseases.

As used herein, the term "kidney disease" means any alteration in normal physiology and function of the kidney. This term includes but is not limited to diseases and conditions such as kidney transplant; nephropathy; primary glomerulopathies, focal segmental glomerulosclerosis including sporadic idiopathic steroid-resistant nephrotic syndrome with focal segmental glomerulosclerosis, Minimal Change disease, Membranous GN, C3 glomerulopathy, gammopathies of renal significance, IgA Nephropathy, chronic kidney disease (CKD); Glomerulonephritis; inherited diseases such as polycystic kidney disease; Acute and chronic interstitial nephritis, Mesoamerican Nephropathy; nephrotic syndrome; Nephritic syndrome, end stage renal disease (ESRD); acute and chronic renal failure; interstitial disease; nephritis; sclerosis, an induration or hardening of tissues and/or vessels resulting from causes that include, for example, inflammation due to disease or injury; renal fibrosis and scarring; renal-associated proliferative disorders; and other primary or secondary nephrogenic conditions.

Kidney diseases may also be generally defined as a "nephropathy" or "nephropathies." The terms "nephropathy" or "nephropathies" encompass all clinical-pathological changes in the kidney which may result in kidney fibrosis and/or glomerular diseases (e.g.. glomerulosclerosis or glomerulonephritis) and/or chronic renal insufficiency, and can cause end stage renal disease and/or renal failure.

Some aspects of the present disclosure relate to compositions and their uses for the prevention and/or treatment of hypertensive nephropathy, diabetic nephropathy such as diabetic kidney disorders, and other types of nephropathy such as analgesic nephropathy, immune-mediated glomerulopathies (e.g., IgA nephropathy or Berger's disease, lupus nephritis), ischemic nephropathy, HIV-associated nephropathy, membranous nephropathy, glomerulonephritis, glomerulosclerosis, radiocontrast media-induced nephropathy, toxic nephropathy, analgesic-induced nephrotoxicity, cisplatin nephropathy, transplant nephropathy, and other forms of glomerular abnormality or injury, or glomerular capillary injury (tubular fibrosis). In some embodiments, the terms "nephropathy" or "nephropathies" refer specifically to a disorder or disease where there is either the presence of proteins (i.e., proteinuria) in the urine of a subject and/or the presence of renal insufficiency, also referred herein as proteinuric kidney disorders.

In some embodiments, the subject is suffering from albuminuria or proteinuria. Exemplary disorders associated with albuminuria include, but are not limited to, chronic kidney disease, proliferative glomerulonephritis (e.g., immunoglobulin A nephropathy, membranoproliferative glomerulonephritis, mesangial proliferative glomerulonephritis, anti-GBM disease, renal vasculitis, lupus nephritis, cryoglobulinemia-associated glomerulonephritis, bacterial endocarditis, Henoch-Schonlein purpura, postinfectious glomerulonephritis, or hepatitis C), and nonproliferative glomerulonephritis (e.g., membranous glomerulonephritis, minimal-change disease, primary focal segmental glomerulosclerosis (FSGS), fibrillary glomerulonephritis, immunotactoid glomerulonephritis, amyloidosis, Alport syndrome, hypertensive nephrosclerosis, light-chain disease from multiple myeloma and secondary focal glomerulosclerosis).

In any of the embodiments provided herein, a subject may be subjected to certain tests to evaluate kidney function. Such tests include, without limitation, measurement of blood urea nitrogen in the subject; measuring creatinine in the blood of the subject; measuring creatinine clearance in the blood of the subject; measuring proteinuria in the subject; measuring albumin: creatinine ratio in the subject; measuring glomerular filtration rate in the subject; and measuring urinary output in the subject.

As used herein, the term "treating" or "treatment", denotes reversing, alleviating, inhibiting the progress of, or preventing the disorder or condition to which such term applies, or reversing, alleviating, inhibiting the progress of, or preventing one or more symptoms of the disorder or condition to which such term applies. Consequently, in another aspect, this disclosure also relates to the compounds of formula I, typically, compounds ***A*** and ***G*** as described in the examples, or their pharmaceutically acceptable salts, for use for diminishing, inhibiting or eliminating the symptoms associated with kidney disorders, including, without limitation, decreasing proteinuria, slowing the increase in proteinuria,

slowing the increase in urinary albumin creatinine ratio (UACR), reducing UACR, slowing the increase in UAER, decreasing UAER, reducing albuminuria, slowing the increase in albuminuria, increasing glomerular podocyte density, preventing or slowing glomerular basement membrane (GBM) thickening, decreasing glomerular area, reducing the number of renal interstitial macrophages, decreasing or slowing fibrosis of renal tissues, stopping or decreasing inflammation in the kidneys, stopping or decreasing macrophage-induced damage to the kidneys, increasing or normalizing estimated glomerular filtration rate (eGFR), attenuating the decline of eGFR, reducing glomerulosclerosis, stopping or decreasing expansion of the glomerular extracellular matrix, stopping or decreasing deposition of hyaline masses, stopping or reducing glomerular epithelial hyperplasia lesions (EPHLs), and stopping or decreasing lymphocyte infiltration.

The disclosure therefore also relates to pharmaceutical compositions comprising a compound of formula **I** as defined above and a pharmaceutically acceptable carrier and/or adjuvant, for use in the treatment of kidney diseases as defined above.

In particular, the disclosure relates to a pharmaceutical composition as defined above for use in the treatment and/or prophylaxis of kidney diseases, including chronic kidney diseases, proteinuric and/or glomerular diseases. Preferred use relate to Alport syndrome, focal segmental glomerulosclerosis, and diabetic kidney diseases.

In another embodiment, the invention relates to a method for the treatment and/or prophylaxis of kidney diseases, which method comprises administering a therapeutically effective amount of a compound of formula **I** to a patient in need thereof. Examples of such kidney diseases include chronic kidney diseases, proteinuric and/or glomerular diseases. A method for the treatment and/or prophylaxis of Alport syndrome, focal segmental glomerulosclerosis, and diabetic kidney diseases is preferred.

In addition, the disclosure relates to the use of compounds of formula **I** as defined above, typically, compounds ***A*** and ***G*** as described in the examples, or their pharmaceutically acceptable salts, for the preparation of a medicament for the treatment and/or prophylaxis of kidney diseases. Examples of such kidney diseases include chronic kidney diseases, proteinuric and/or glomerular diseases. The use of compounds of formula **I** as defined above for the preparation of medicaments for the treatment and/or prophylaxis of Alport syndrome, focal segmental glomerulosclerosis, and diabetic kidney diseases is of particular interest.

The form of the pharmaceutical compositions, the route of administration, the dosage and the regimen naturally depend upon the condition to be treated, the severity of the illness, the age, weight, and sex of the patient, etc.

The pharmaceutical compositions of the disclosure can be formulated for a topical, oral, intranasal, intraocular, intravenous, intramuscular or subcutaneous administration and the like. Preferably, the pharmaceutical compositions of the disclosure can be formulated for an oral administration.

In a specific embodiment, the pharmaceutical compositions of the disclosure can be formulated for an intravitreal, intrathecal or transdermal administration.

The pharmaceutical compositions can take the form of tablets, pills, capsules, semisolids, powders, sustained release formulations, solutions, suspensions, emulsions, syrups, elixirs, aerosols, or any other appropriate compositions; and comprise at least one compound of formula I as defined above.

In a specific embodiment, an oral formulation is a tablet, an orodispersible tablet, a capsule, solution, patch, a sublingual tablet, a nasal spray or an oral spray. In one sub-embodiment, the formulation is prepared for sustainable release of the compound of formula I as defined above.

Because of their ease in administration, tablets and capsules represent the most advantageous oral dosage unit form, in which case solid pharmaceutical carriers are obviously employed. If desired, tablets may be sugar coated or enteric coated by standard techniques. The tablets or pills can be coated to provide a dosage form affording the advantage of prolonged action. For example, the tablet or pills can comprise an inner dosage and an outer dosage component, the latter being in the form of an envelope over the former. The two components can be separated by an enteric layer, which serves to resist disintegration in the stomach and permits the inner component to pass intact into the duodenum or to be delayed in release. A variety of material can be used for such enteric layers or coatings, such materials including a number of polymeric acids with such materials as shellac, cetyl alcohol and cellulose acetate.

Suitable carrier materials are not only inorganic carrier materials, but also organic carrier materials. Thus, for example, lactose, corn starch or derivatives thereof, talc, stearic acid or its salts can be used as carrier materials for tablets, coated tablets, dragées and hard gelatine capsules. Suitable carrier materials for soft gelatine capsules are, for example, vegetable oils, waxes, fats and semi-solid and liquid polyols (depending on the nature of the active ingredient no carriers might, however, be required in the case of soft gelatine capsules). Suitable carrier materials for the production of solutions and syrups are, for example, water, polyols, sucrose, invert sugar and the like. Suitable carrier materials for injection solutions are, for example, water, alcohols, polyols, glycerol and vegetable oils. Suitable carrier materials for suppositories are, for example, natural or hardened oils, waxes, fats and semi-liquid or liquid polyols. Suitable carrier materials for topical preparations are glycerides, semi-synthetic and synthetic glycerides, hydrogenated oils, liquid waxes, liquid paraffins, liquid fatty alcohols, sterols, polyethylene glycols and cellulose derivatives.

Usual stabilizers, preservatives, wetting and emulsifying agents, consistency-improving agents, flavor-improving agents, salts for varying the osmotic pressure, buffer substances, solubilizers, colorants and masking agents and antioxidants come into consideration as pharmaceutical adjuvants.

The doses used for the administration can be adapted as a function of various parameters, and in particular as a function of the mode of administration used, of the relevant pathology, or alternatively of the desired duration of treatment. It will be appreciated that appropriate dosages of the compounds, and compositions comprising the compounds, can vary from patient to patient. Determining the optimal dosage will generally involve the balancing of the level of therapeutic benefit against any risk or deleterious side effects of the treatments described herein. The selected dosage level will depend on a variety of factors including, but not limited to, the activity of the particular compound, the route of administration, the time of administration, the rate of excretion of the compound, the duration of the treatment, other drugs, compounds, and/or materials used in combination, and the age, sex, weight, condition, general health, and prior medical history of the patient. The amount of compound and route of administration will ultimately be at the discretion of the physician, although generally the dosage will be to achieve local concentrations at the site of action which achieve the desired effect without causing substantial harmful or deleterious side-effects.

For adult patients a daily dosage of about 1 to 100 mg, especially about 1 to 50 mg, comes into consideration. Depending on severity of the disease and the precise pharmacokinetic profile the compound could be administered with one or several daily dosage units, e.g. in 1 to 3 dosage units.

In a particular embodiment, the compound for use according to the present disclosure is administrated with a daily dose of 20 to 800 mg/day.

In another particular embodiment, the compound for use according to the present disclosure is administrated with a daily dose of 200 mg/day.

The pharmaceutical compositions for use according to the present disclosure conveniently contain about 1-200 mg, preferably 75-100 mg, of a compound of formula I, typically, compounds ***A*** and ***G*** as described in the examples, or their pharmaceutically acceptable salts. The dosing regimen might be tailored to the specific pharmacokinetic properties of a compound of formula I.

In a particular embodiment, the coumpound of formula I, typically, compounds ***A*** and ***G*** as described in the examples, or pharmaceutical compositions comprising thereof, is administrated once daily, twice daily, three time a day, once every three days, once weekly, once every two weeks, or once monthly. Preferably, the dosing periodicity is selected from twice per day, once per day and once every othe day.

In another particular embodiment, the loading dose regimen of a compound of formula I, typically, compounds ***A*** and ***G*** as described in the examples, or their pharmaceutically acceptable salts, double the dose for the first 7 days, 14 days, and 30 days.

In another particular embodiment, the pharmaceutical compositions for use according to the present disclosure may contain about 20-800 mg, preferably about 50-400 mg, and more preferably of about 200 mg, of a compound of formula I, typically, compounds ***A*** and ***G*** as described in the examples, or their pharmaceutically acceptable salts.

In another particular embodiment, for use according to the present disclosure, the compound of formula I, typically, compounds ***A*** and ***G*** as described in the examples, or their pharmaceutically acceptable salts, the daily dose is to 20 to 800 mg/day, preferably the daily dose is about 200 mg/day.

In addition, the compounds of formula I for use according to the present disclosure may also be useful in combination or association, simultaneously, separately or sequentially with another agent for preventing or treating a kidney disease, or an associated disorder or complication. Examples of such known compounds include but are not limited to: angiotensin-converting enzyme (ACE) inhibitor drugs (e.g., captopril (Capoten®), enalapril (Innovace®), fosinopril (Staril®), lisinopril (Zestril®), perindopril (Coversyl®), quinapril (Accupro®), trandanalopril (Gopten®), lotensin, moexipril, ramipril); RAS blockers; angiotensin receptor blockers (ARBs) (e.g., Olmesartan, Irbesartan, Losartan, Valsartan, candesartan, eprosartan, telmisartan, etc); protein kinase C (PKC) inhibitors (e.g., ruboxistaurin); inhibitors of AGE-dependent pathways (e.g., aminoguanidine, ALT-946, pyrodoxamine (pyrododorin), OPB-9295, alagebrium); anti-inflammatory agents (e.g., clyclooxigenase-2 inhibitors, mycophenolate mophetil, mizoribine, pentoxifylline), GAGs (e.g., sulodexide (U.S. Pat. No. 5,496,807)); pyridoxamine (U.S. Pat. No. 7,030,146); endothelin antagonists (e.g., SPP 301), COX-2 inhibitors, PPAR-$\gamma$ antagonists and other compounds like amifostine (used for cisplatin nephropathy), captopril (used for diabetic nephropathy), cyclophosphamide and rituximab (used for idiopathic membranous nephropathy), sodium thiosulfate (used for cisplatin nephropathy), tranilast, cyclodextrins and their derivatives (e.g. hydroxypropyl-beta-cyclodextrin), etc. (Williams and Tuttle (2005), Advances in Chronic Kidney Disease, 12 (2):212-222; Giunti et al. (2006), Minerva Medica, 97:241-62). In specific embodiments, known compounds for use in combination or association, include without limitation, Bardoxolone or oligonucleotide inhibitor of mir-21 (mir-21 antagomir).

In another specific embodiment, known compounds for use in combination or association include vitamin D derivatives, anti-hyperglycemic agents (e.g., SGLT2 inhibitors, GLP1 agonist, DPP4 inhibitors), anti-hypercholesterolemic agents (e.g., statin, niacin, fibrates, PCSK9 inhibitors, ezetimibe).

As used herein, the term "combination" refers to either a fixed dose combination in one unit dosage form, non-fixed dose combination, or a kit of parts for the combined administration where a compound of formula I, and one or more

combination partners (e.g. an ACE inhibitor drug or ARB drug) may be administered independently at the same time or separately within time intervals, especially where these time intervals allow the combination partners show a cooperative, e.g. synergistic effect.

The term "fixed dose combination" means that the active ingredients are both administered to a patient simultaneously in the form of a single entity or dosage.

The term "non-fixed dose combination" means that the active ingredients, e.g. a compound of formula I and one or more combination partners (e.g. an ACE inhibitor drug or ARB drug), are both administered to a patient as separate entities either simultaneously or sequentially, with no specific time limits, wherein such administration provides therapeutically effective levels of the two compounds in the body of the patient.

Additionally, the methods described herein may also include co-administration of at least one other therapeutic agent for the treatment of another disease directly or indirectly related to kidney disease complications, including but not limited to: dyslipidemia, hypertension, obesity, neuropathy, inflammation, and/or retinopathy. Such additional therapeutic agents include, but are not limited to, corticosteroids; immunosuppressive medications; antibiotics; antihypertensive and diuretic medications (such as thiazide diuretics and ACE-inhibitors or β-adrenergic antagonists); lipid lowering agents such as bile sequestrant resins, cholestyramine, colestipol, nicotinic acid, and more particularly drugs and medications used to reduce cholesterol and triglycerides (e.g., fibrates (e.g., Gemfibrozil®) and HMG-CoA inhibitors such as Lovastatin®, Atorvastatin®, Fluvastatin®, Lescol®, Lipitor®, Mevacor®, Pravachol®, Pravastatin®, Simvastatin®, Zocor®, Cerivastatin®, etc); nicotinic acid; and Vitamin D.

The terms "co-administration" or "combined administration" as used herein, are meant to encompass administration of the selected combination partner to a single subject therein, and are intended to include treatment regimens in which the agents are not necessarily administered by the same route of administration or at the same time.

The term "jointly therapeutically effective" means that the therapeutic agents may be given separately (in a chronologically staggered manner, especially a sequence-specific manner) in such time intervals to show a (preferably synergistic) interaction (i.e. joint therapeutic effect).

The disclosure therefore also relates to a medicament comprising

(i) a compound of formula **I** as defined above, typically, compound ***A*** or ***G*** as described in the Examples, in combination or association with

(ii) a compound selected from the group consisting of angiotensin-converting enzyme (ACE) inhibitor drugs; RAS blockers; angiotensin receptor blockers (ARBs); protein kinase C (PKC) inhibitors; inhibitors of AGE-dependent pathways; anti-inflammatory agents, GAGs; pyridoxamine (U.S. Pat. No. 7,030,146); endothelin antagonists, COX-2 inhibitors, PPAR-γ antagonists and other compounds like amifostine (used for cisplatin nephropathy), captopril (used for diabetic nephropathy), cyclophosphamide (used for idiopathic membranous nephropathy), sodium thiosulfate (used for cisplatin nephropathy), or tranilast ; cyclodextrins and their derivatives (e.g. hydroxypropyl-beta-cyclodextrin), and,

(iii) a pharmaceutically acceptable carrier and/or adjuvant.

As used herein, the term "medicament" means a pharmaceutical composition, or a combination of several pharmaceutical compositions, which contains one or more active ingredients in the presence of one or more excipients.

The disclosure further relates to compounds of formula **I** as defined above for the simultaneous, sequential or separate use with a compound selected from the group consisting of angiotensin-converting enzyme (ACE) inhibitor drugs; RAS blockers; angiotensin receptor blockers (ARBs); protein kinase C (PKC) inhibitors; inhibitors of AGE-dependent pathways; anti-inflammatory agents, GAGs; pyridoxamine (U.S. Pat. No. 7,030,146); endothelin antagonists, COX-2 inhibitors, PPAR-γ antagonists and other compounds like amifostine (used for cisplatin nephropathy), captopril (used for diabetic nephropathy), cyclophosphamide (used for idiopathic membranous nephropathy), sodium thiosulfate (used for cisplatin nephropathy), tranilast or cyclodextrins and their derivatives (e.g. hydroxypropyl-beta-cyclodextrin), vitamin D derivatives, anti-hyperglycemic agents and anti-hypercholesterolemic agents.The disclosure also relates to a method for the treatment and/or prophylaxis of kidney diseases, which method comprises administration of a therapeutically effective amount of a compound according to formula I in combination or association with a therapeutically effective amount of a compound selected from the group consisting of angiotensin-converting enzyme (ACE) inhibitor drugs; RAS blockers; angiotensin receptor blockers (ARBs); protein kinase C (PKC) inhibitors; inhibitors of AGE-dependent pathways; anti-inflammatory agents, GAGs; pyridoxamine (U.S. Pat. No. 7,030,146); endothelin antagonists, COX-2 inhibitors, PPAR-γ antagonists and other compounds like amifostine (used for cisplatin nephropathy), captopril (used for diabetic nephropathy), cyclophosphamide (used for idiopathic membranous nephropathy), sodium thiosulfate (used for cisplatin nephropathy), tranilast, or cyclodextrins and their derivatives (e.g. hydroxypropyl-beta-cyclodextrin), vitamin D derivatives, anti-hyperglycemic agents and anti-hypercholesterolemic agents.

In the following examples, compounds of formula (I) have been tested in particular in in vivo animal models of at least 3 distinct kidney diseases, namely, Alport syndrome, focal segmental glomerulosclerosis and diabetic kidney disorders, reflecting a promising therapeutic effect of such compounds in a number of kidney diseases.

## LEGENDS OF THE FIGURES

**Figure 1. Experimental design to test optimal dose of the compounds in ADR-induced nephropathy in Balb/c mice.** 30 female Balb/c mice were injected with Doxorubin (ADR, adriamycin) at a dose of 12 mg/kg via the tail vein injection. A baseline group of 5 mice received saline solution. ADR-injected mice were then separated into 6 groups of five animals each, yielding seven total experimental groups. Starting the next day, compounds were administrated once daily by oral gavage for 5 weeks. Urines were collected weekly and body weight was recorded weekly. Blood and kidney cortexes were collected 35 days post ADR-injection at sacrifice.

**Figure 2: Experimental design to test the compounds in ADR-induced nephropathy in Balb/c mice.** Female Balb/c mice were injected with Doxorubin (ADR, adriamycin) at a dose of 12 mg/kg via the tail vein injection. A control group of 5 mice received saline solution. ADR injected females were separated into five groups of 6. Starting the next day, vehicle or compounds were administrated once daily by oral gavage for 4 weeks as indicated. Urines were collected weekly and body weight was recorded weekly. Blood and kidney cortexes were collected 28 days post-ADR-injection at sacrifice.

**Figure 3. Compound cytotoxicity in differentiated human podocytes.** Differentiated human podocytes were treated with 0 μM (vehicle), 1 μM, 5 μM and 10 μM of Cpd A, Cpd C, Cpd D, Cpd E, Cpd F and Cpd G for 18 h. Cytotoxicity was assessed using the Promega CytoTox Assay kit. Treatment with plain medium (open bars) was used as baseline. Cytotoxicity signal was normalized to viability signal to eliminate bias due to differences in cell number. All treatments were compared to the vehicle-treated cells. Only significant toxicity was found at concentrations above 10 uM of Cpd A and Cpd C. One-way-ANOVA, n=3 independent experiments, Dunett test, $*p<0.5$, $***p<0.001$.

**Figure 4. Treatment with Cpd C, Cpd A and Cpd G increases the ABCA1 expression and cholesterol efflux in differentiated podocytes.**
Differentiated human podocytes were treated with vehicle (0.1 % DMSO) or compounds as indicated for 18 h and ABCA1 expression, localization and participation in cholesterol efflux was measured, **(a)** ABCA1 and GAPDH western blot in whole cell lysates showing strong induction of ABCA1 expression with LXR agonists (Cpds C, E and D) and modest induction with 10 μM ABCA1 inducers Cpds A and G. **(b-e)** Cpd C, Cpd A and Cpd G increase the expression of ABCA1 at the plasma membrane. Differentiated human podocytes treated with vehicle or compounds C, A and G for 18 h as indicated were subjected to cell fractionation. **(b)** ABCA1 and GAPDH western blot in whole cell lysates showing modest increase of ABCA1 expression after treating with Cpd A (5uM) and Cpd G (10 uM). (c-e) Plasma membrane, microsomal and cytosolic collected fractions blotted and probed for ABCA1, $Na^+/K^+$ ATPase pump and MEK. **(f-h)** Cpd C, A and G increase ApoA1-mediated cholesterol efflux in differentiated podocytes. Differentiated podocytes loaded with [$^3$H]-cholesterol were incubated for 18 h with compounds as indicated. ApoA1-mediated cholesterol efflux was calculated after incubating cells with or without ApoA1 for 18h. Data reported as the mean of at least three independent experiments with s.d. One-way ANOVA, Dunnett's test, $*P<0.05\%$, $**P<0.01\%$

**Figure 5. Selecting best dose of compounds to attenuate kidney damage induced by ADR.**

**(a-e)** Albuminuria in ADR injected mice during a 5 week-treatment with cpds at indicated doses. Mice develop massive proteinuria 2-3 weeks following ADR injection, which was attenuated in mice treated with a dose of 30 mg/Kg of Cpd A **(b)** and 100 mg/Kg of Cpd G **(e).**

**(f-j).** ADR-injected mice are characterized by a significant drop of body weight two to three weeks following ADR injections, a phenotype that is partially attenuated in mice that received 30 mg/kg of Cpd A or 100 mg/kg of Cpd G. Results shown represent the mean and SE of each group. One-way ANOVA n=5, Dunett test, $*P<0.05\%$.

**Figure 6. ABCA1 inducers Cpd A and Cpd G significantly reduce albuminuria and body weight loss in mice injected with ADR.** Mice injected with ADR (12 mg/Kg) received vehicle, LXR agonist Cpd C or optimal doses of ABCA1 inducers, Cpd A and Cpd G, once daily for 28 days. Albuminuria and body weight was measured once weekly, **(a)** Albuminuria after a 4 week-treatment. **(b)** Weight loss, expressed as the difference between the weight each mice

had after 4 weeks of treatment and one day before ADR injection. The baseline group, mice not injected with ADR, shown at left, reflect the phenotype without kidney damage. Bars represent the median and the range of each treatment group. All groups were compared to the one that received vehicle using Mann Whitney test (n=8, *P<0.05%, **P< 0.01%, ****P<0.0001%).

**Figure 7.** Pathological exam of PAS and HE sections from kidneys of animals injected with ADR and treated with vehicle or 100 mg/Kg/day of Cpd G for 4 weeks. Pathologist evaluated the % of global sclerosis **(A);** % segmental sclerosis **(B);** Podocyte hypertrophy **(C);** Podocyte Hyperplasia **(D);** Tubular microcysts **(E)** and Interstitial inflammation **(F).** Scale values represent the following: 0: 0 %; 0.5+: 1-10 %; 1+: 11-25%; 2+: 26-50 %; 3+: 51-75 %; 4+ > 75%. Samples from Cpd G-treated group were compared to the vehicle treated using Mann Whitney test (n=7; * P < 0.05%)

**Figure 8.** Mice injected with saline solution or ADR were treated with either vehicle or 100 mg/Kg of Cpd G for 28 days. Kidney cortex sections were stained with ORO to detect lipid droplet deposition. Representative pictures of sections from healthy baseline group (A), injected with ADR and treated with vehicle (B) and 100 mg/Kg/day of Cpd G (C).

**Figure 9. Cpd G reduces accumulation of esterified cholesterol in renal tissue of ADR-injected mice.** Lipids extracted from kidney cortex of ADR-injected mice that were treated with vehicle or Cpd G for 28 days were assayed for cholesterol esters, total cholesterol and triglycerides. The amount of each lipid species was normalized to the total proteins present in the specimen. **(a)** Esterified cholesterol, **(b)** total cholesterol content and **(c)** triglyceride content found in renal tissue. Animals that did not received ADR injection are shown on the left and represent baseline values when no kidney injury is induced. Groups treated with Cpd G and vehicle were compared using Mann Whitney double tailed test, n=8 ; **** P<0.0001%. **(d-f).** Correlation between the albuminuria of each mouse and the amount of lipid species present in its kidney cortex. A strong correlation was found with cholesterol esters **(d),** but not with total cholesterol (e) and triglycerides **(f).** Pearson test, n=10

**Figure 10.** Animals injected with 12 mg/Kg of ADR were treated with vehicle or 100 mg/Kg of Cpd one day following ADR injection. Picture taken after 20 days of treatment with vehicle **(A-B)** and 100 mg/Kg of Cpd G **(C-D)**

**Figure 11. Treatment of Col4A3 KO mice with Cpd G delays progression to end stage renal disease.** 129-Col4A3 KO mice of 4 weeks age were treated with vehicle or 100 mg/Kg of Cpd G for 4 weeks. At the end of the experiment, 56 days, body weight was measured, spot urine and blood were collected and kidneys were analyzed for lipid accumulation, **(a)** Albuminuria, **(b)** serum creatinine, **(c)** blood urea nitrogen and **(d)** body weight of KO mice treated with vehicle or Cpd G. **(e)** Representative pictures of PAS-stained kidney sections from each group, **(f)** Mesangial expansion quantification after a blind pathological analysis of PAS-stained kidney sections mentioned in **e.** Col4a3+/+ mice (wt) of the same age, are included on the left to reflect the phenotype in the absence of chronic kidney disease. **(g)** Survival curve of 129-Col4a3 KO mice that received vehicle or Cpd G (100mg/Kg/day) for 4 weeks starting at the age of 6 weeks. The horizontal bars represent the median of each group. Statistical differences between the groups were calculated using a double-tailed Mann Whitney test, n=4, * P< 0.05 %, **P<0.01 %.

**Figure 12.** Db/+, db/db vehicle treated and db/db ABCA1 inducer (compound A) treated mice were utilized and analyzed for: albumin to creatinine ratios determined at start of treatment (14 weeks), after 2 weeks of treatment (16 weeks) and at time of sacrifice after 4 weeks of treatment (18 weeks) in **(A);** blood urea nitrogen (BUN) determined from mouse serum and presented in mg/dL **(B);** kidney cortex cholesterol content (fold change in nmol of cholesterol per mg of protein) in forms of total cholesterol (TC), free cholesterol (FC) and cholesterol esters (CE) **(C);** correlation between BUN and CE **(D);** podocyte number per glomerular cross section determined via WT1 antibody **(E)** and quantified **(F);** mesangial expansion score using PAS stained kidney cortex sections **(G)** and quantified **(H);** podocyte foot process effacement determined from TEM images **(I)** and quantified **(J).** One-way ANOVA followed by Tukey's test *p<0.05, **p<0.01, ***p<0.001, ****p<0.0001.

**Figure 13. Cpd G reduces esterified cholesterol accumulation in kidney cortexes (a)** Esterified cholesterol (CE), **(b)** total cholesterol (TC) and **(c)** triglyceride content (TG) was determined in kidney cortexes from 8-week old Col4A3KO that received vehicle or Cpd G for 28 days starting at the age of 4 weeks. The content of each lipid was normalized to the total protein content. A group of wildtype littermates was also included in this study (open circles). The horizontal bars represent the median of each group. Statistical differences were determined using a double tailed Mann Whitney test (n=8), *P<0.05, **P<0.001.

## EXAMPLES

***Preparation of ABCA1 inducer compounds***

**List of compounds:**

| | Chemical name | Structure |
|---|---|---|
| ***G*** | 5-(3,4-dichloro-phenyl)-*N*-((1*R*,2*R*)-2-hydroxy-cyclohexyl)-6-(2,2,2-tri-fluoro-ethoxy)-nicotinamide | 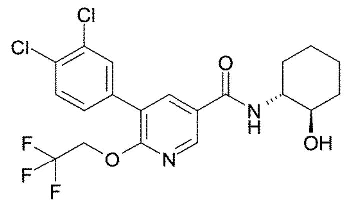 |
| ***A*** | 6-(3,4-Dichlorophenyl)-*N*-[(1*R*,2*R*)-2-hydroxycyclohexyl]-5-(2,2,2-tri-fluoroethoxy)pyridine-2-carboxamide | 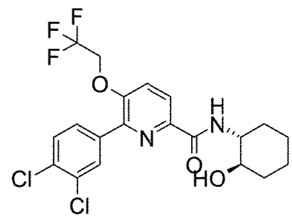 |
| ***F*** | 6-(4-chlorophenyl)-N-(pyrimidin-5-yl)-5-(2,2,2-trifluoroethoxy)picolina-mide | 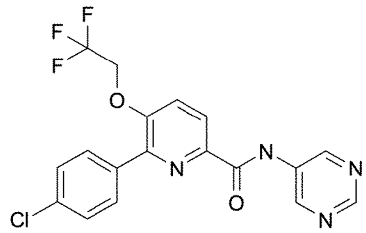 |

The above compounds and their synthesis methods have been described in WO2014/180741.

**Preparation of Compound *G* (5-(3,4-dichloro-phenyl)-*N*-((1*R*,2*R*)-2-hydroxycyclohexyl)-6-(2,2,2-trifluoro-ethoxy)-nicotinamide)**

The compound was prepared from 5-bromo-6-chloro-3-pyridinecarboxylic acid, 2,2,2-trifluoroethanol, (1*R*,2*R*)-2-amino-cyclohexanol and 3,4-dichlorophenylboronic acid in accordance with the procedure described in WO 2011/029827, Example 3. MS 463.079, 465.077 $(M+H)^+$.

**Preparation of Compound *A* 6-(3,4-Dichlorophenyl)-*N*-[(1*R*,2*R*)-2-hydroxycyclohexyl]-5-(2,2,2-trifluoroethoxy) pyridine-2-carboxamide**

Compound A was prepared in accordance with the procedure described in WO 2014/180741 in the following steps:

a) 6-Chloro-2-(3,4-dichlorophenyl)-3-fluoropyridine
In a 100 mL four-necked flask, 2,6-dichloro-3-fluoropyridine (765 mg, 4.61 mmol, Eq: 1.00, CAS Reg. No. 52208-50-1) and potassium (3,4-dichlorophenyl)trifluoroborate (1.21 g, 4.61 mmol, Eq: 1.00, CAN 850623-68-6) were combined with dioxane (23 mL) and water (13 mL). 2M $Na_2CO_3$ (6.91 mL, 13.8 mmol, Eq: 3) was added, followed by $PdCl_2$ (DPPF)-$CH_2Cl_2$ adduct (169 mg, 230 μmol, Eq: 0.05, CAS Reg. No. 95464-05-4). The reaction mixture was 3x degassed and purged with argon and then heated under stirring overnight to 60°C. The reaction mixture was cooled to ambient temperature, poured into 50 mL $H_2O$ and extracted with tert. butyl-methyl-ether (2x100mL). The organic layers were washed with $H_2O$/brine, combined, dried over $Na_2SO_4$ and concentrated in vacuo. The crude material was purified twice by flash chromatography (silica gel, 70g, 5% to 20% dichloromethane in heptane) to yield 540mg of

the title compound as white semisolid.

b) 6-Chloro-2-(3,4-dichlorophenyl)-3-(2,2,2-trifluoroethoxy)pyridine
In a 25 mL pear-shaped flask, the above prepared 6-chloro-2-(3,4-dichlorophenyl)-3-fluoropyridine (530 mg, 1.44 mmol, Eq: 1.00) was combined with DMSO (8 mL). KOH (118 mg, 2.1 mmol, Eq: 1.46) and 2,2,2-trifluoroethanol (211 mg, 153 μl, 2.11 mmol, Eq: 1.47) were added and the reaction allowed to proceed for 2h at ambient temperature. The mixture was poured into 30mL $H_2O$ and extracted with tert. butyl-methyl-ether (2x40mL). The organic layers were washed with $H_2O$/brine, combined, dried over $Na_2SO_4$ and concentrated in vacuo. The crude product was purified by flash chromatography (silica gel, 70g, 5% to 20% EtOAc in heptane) to yield 444mg of the title compound as colorless liquid; MS (ESI) 356.3, 358.3, 360.3 $(M+H)^+$.

c) Methyl 6-(3,4-dichlorophenyl)-5-(2,2,2-trifluoroethoxy)pyridine-2-carboxylate
In a 35 mL autoclave the above prepared 6-chloro-2-(3,4-dichlorophenyl)-3-(2,2,2-trifluoroethoxy)pyridine (437 mg, 1.22 mmol, Eq: 1.00) was dissolved in 5 mL MeOH. Protected from oxygen and moisture by Argon, $PdCl_2$ (DPPF)-$CH_2Cl_2$ adduct (75.2 mg, 92 μmol, Eq: 0.083, CAS Reg. No. 95464-05-4) was added, followed by triethylamine (233 mg, 321 mL, 2.31 mmol, Eq: 2.31). The reactor was then flushed three times with CO, pressurized to 70 bar, and the carbonylation then allowed to proceed for 20 h at 110 °C. After cooling and release of the pressure, the crude reaction mixture was concentrated in vacuo. The residue was then redissolved in AcOEt/$H_2O$ and transferred to a separatory funnel. The aqueous layer was back-extracted with EtOAc, the organic layers were washed with $H_2O$ and brine, combined, dried over $Na_2SO_4$ and concentrated in vacuo. Flash chromatography (silica gel, 70g, 10% to 40% EtOAc in heptane) delivered finally 327 mg of the title product as white solid; MS (ESI) 380.4, 382.4 $(M+H)^+$.

d) 6-(3,4-Dichlorophenyl)-5-(2,2,2-trifluoroethoxy)pyridine-2-carboxylic acid
In a 25 mL pear-shaped flask, the above prepared methyl 6-(3,4-dichlorophenyl)-5-(2,2,2-trifluoroethoxy)picolinate (326 mg, 858 μmol, Eq: 1.00) was combined with tetrahydrofuran (5 mL) to give a colorless solution. Water (2.5 ml) was added, followed by LiOH (41.1 mg, 1.72 mmol, Eq: 2) and the reaction mixture was stirred for 2hr at 40 °C. The reaction mixture was poured into 5mL sat. $NH_4Cl$ sol. and 3mL 1N $KHSO_4$ sol. and extracted with EtOAc (2 x 30 mL).The organic layers were combined, dried over $Na_2SO_4$ and concentrated in vacuo to leave 322 mg of the title acid as white foam; MS (ESI) 366.4, 368.4 $(M+H)^+$.

e) 6-(3,4-Dichlorophenyl)-*N*-[(1R,2R)-2-hydroxycyclohexyl]-5-(2,2,2-trifluoroethoxy)pyridine-2-carboxamide
In a 25 mL pear-shaped flask, the above synthesized 6-(3,4-dichlorophenyl)-5-(2,2,2-trifluoroethoxy)-picolinic acid (322 mg, 879 μmol, Eq: 1.00) was dissolved in DMF (12 mL). TBTU (O-(benzotriazol-1-yl)-*N,N,N',N'*-tetramethyluronium tetrafluoroborate, 424 mg, 1.32 mmol, Eq: 1.5, CAS Reg. No. 125700-67-6) and *N,N*-diisopropylethylamine (568 mg, 768 μl, 4.4 mmol, Eq: 5) were added and the reaction mixture stirred for 10min at ambient temperature before (1R,2R)-2-aminocyclohexanol hydrochloride (160 mg, 1.06 mmol, Eq: 1.2, CAS Reg. No. 13374-31-7) was added without additional solvent. The reaction was then allowed to proceed for 3 h at room temperature. The crude mixture was diluted with $H_2O$ and extracted with $CH_2Cl_2$. The organic layers were combined, dried over $Na_2SO_4$ and concentrated in vacuo. Purification by flash chromatography (basic alumina, 50g, 10% to 80% EtOAc in heptane) and precipitation of the crude product from EtOAc and heptane afforded the title amide as white solid; high resolution MS (ESI) 463.0798, 465.0769 $(M+H)^+$; expected: 463.0798, 465.0768.

**Preparation of Compound *H* (N'-(6-chloropyridazin-3-yl)-5-(4-cyanophenyl)-N'-methyl-6-(2,2,2-trifluoroethoxy) pyridine-3-carbohydrazide)**

Compound H was prepared in accordance with the procedure described in WO 2014/180741 in the following steps:

a) 5-(4-Cyano-phenyl)-6-(2,2,2-trifluoro-ethoxy)-nicotinic acid methyl ester
In a 50 mL 4-necked flask, methyl 5-bromo-6-(2,2,2-trifluoroethoxy)nicotinate (1 g, 3.18 mmol, Eq: 1.00, CAS Reg. No. 1211589-51-3) and cesium carbonate (3.11 g, 9.55 mmol, Eq: 3) were combined with toluene (25 mL) and water (2.8 mL) to give a colorless solution. The reaction mixture was 3x degassed and purged with argon; then palladium(II) acetate (14.3 mg, 63.7 μmol, Eq: 0.02), potassium (4-cyanophenyl)trifluoroborate (732 mg, 3.5 mmol, Eq: 1.1, CAS Reg. No. 850623-36-8) and butyldi-1-adamantylphosphine (68.5 mg, 191 μmol, Eq: 0.06, CAS Reg. No. 321921-71-5) were successively added. The degassing-purging cycle was repeated after each addition. The reaction mixture was then heated to 120 °C for 5 hours. After cooling, the reaction mixture was poured onto 50mL $H_2O$ and extracted with AcOEt (2x50mL). The organic layers were washed with $H_2O$/brine, combined, dried over $Na_2SO_4$ and

concentrated in vacuo. Purification by flash chromatography (silica gel, 50 g, 50% to 100 % $CH_2Cl_2$ in heptane) yielded finally 898mg of the title compound as white foam; MS (ESI) 337.2 $(M+H)^+$.

b) 5-(4-Cyano-phenyl)-6-(2,2,2-trifluoro-ethoxy)-nicotinic acid
In a 25 mL round-bottomed flask, the above prepared 5-(4-cyano-phenyl)-6-(2,2,2-trifluoro-ethoxy)-nicotinic acid methyl ester (0.891 g, 2.65 mmol, Eq: 1.00) was combined with THF (7 mL) and water (3.5 mL) to give a light yellow biphasic system. Lithium hydroxide (127 mg, 5.3 mmol, Eq: 2) was added and the reaction mixture was stirred at 40 °C for 3 hours when TLC indicated the reaction to be complete. Work up: 10mL $H_2O$ and 7 mL HCl 1N were added, the mixture extracted with AcOEt (2x50mL), the organic layers were combined, washed with brine, dried over $Na_2SO_4$ and concentrated in vacuo. Trituration with heptane / EtOAc 9:1 afforded finally 794 mg of the desired title product as white solid; MS (ESI) 321.2 $(M-H)^-$.

c) *N'*-('6-chloropyridazin-3-yl)-5-('4-cyanophcnyl)-*N'*-methyl-6-(2,2,2-trifluoroethoxy)pyridine-3-carbohydrazide
In a 5mL round-bottomed flask, the above prepared 5-(4-cyano-phenyl)-6-(2,2,2-trifluoro-ethoxy)-nicotinic acid (0.050 g, 155 μmol, Eq: 1.00) was combined with THF (2 mL) to give a colorless solution. TBTU (O-(benzotriazol-1-yl)-*N,N,N',N'*-tetramethyluronium tetrafluoroborate, 74.7 mg, 233 μmol, Eq: 1.5, CAS Reg. No. 125700-67-6) and N,N-diisopropylethylamine (100 mg, 135 μL, 776 μmol, Eq: 5) were added. The reaction mixture was stirred for 10min at RT, then 3-chloro-6-(1-methylhydrazinyl)-pyridazine (29.5 mg, 186 μmol, Eq: 1.2, CAN 76953-33-8) was added and the reaction mixture kept at RT overnight. Pouring into 25 mL 1 M HCl, extraction with EtOAc (2 x 50 mL), washing with 1 M NaOH, drying over $Na_2SO_4$ and evaporation of all solvents in vacuo, followed by flash chromatography (silica gel, 10 g, 2% to 10 % MeOH in $CH_2Cl_2$) and crystallization from heptane / AcOEt generated eventually 28mg of the title compound as white solid; MS (ESI) 463.1, 465.3 $(M+H)^+$.

**Preparation of Compound *J* (6-(4-Chloro-phenyl)-5-(2,2,2-trifluoro-ethoxy)-pyridine-2-carboxylic acid (3-isopropyl-isoxazol-5-ylmethyl)-amide)**

Compound J was prepared in accordance with the procedure described in WO 2014/180741 in the following steps:
The title compound was synthesized from 6-(4-chloro-phenyl)-5-(2,2,2-trifluoro-ethoxy)-2-pyridine carboxylic acid and 3-(1-methylethyl)-5-isoxazolemethanamine (CAS Reg. No. 543713-30-0) in accordance with the method described in WO 2012/032018, Example 64. LC-MS (UV peak area/ESI) 100.0%, 454.4 $(M+H)^+$.

**Preparation of Cpd *F* (6-(4-chlorophenyl)-N-(pyrimidin-5-yl)-5-(2,2,2-trifluoroethoxy)picolinamide, Comparative Compound):**

6-(4-Chloro-phenyl)-5-(2,2,2-trifluoro-ethoxy)-pyridine-2-carboxylic acid (prepared as described in WO 2012/032018, Example AE) was combined with DMF (30 ml) at RT, to give a colorless solution. Pyrimidin-5-amine, TBTU and N-Ethyldiisopropylamine were added. The reaction mixture was stirred at RT for 15 h. The reaction mixture was poured into 150 mL $H_2O$ and extracted with EtOAc (2 x 150 mL). The combined organic layers were washed with brine, dried over $MgSO_4$ and evaporated. The crude material was purified by flash chromatography (silica gel, 20g, 0% to 50% EtOAc in hexane). LC-MS (ESI) 409.068 $(M+H)^+$.

***Comparative other Compounds***

| | Chemical name | Structure |
|---|---|---|
| ***C*** | 1,1,1,3,3,3-Hexafluoro-2-{2-methyl-1-[5-methyl-2-(3-trifluoromethyl-phenyl)-oxazo1-4-ylmethyl]-1H-indo1-5 -yl} -propan-2-ol | 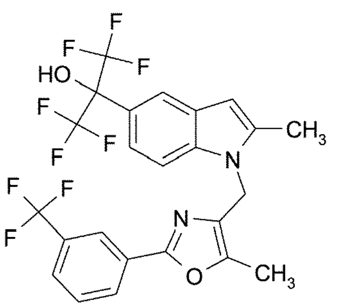 |

(continued)

| | Chemical name | Structure |
|---|---|---|
| ***D*** | 4- {5-[(RS)-(3-Bromobenzenesulfonyl)-((SR)-7-chloro-1,2,3,4-tetrahydro-cyclopenta[b]indol-2-yl)-fluoromethyl]-[1,3,4]oxadiazol-2-yl}-benzoic acid | 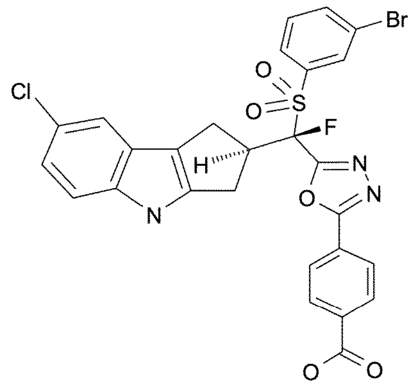 |
| ***E*** | (R)-2-[(S)-Benzenesulfonyl-fluoro-(5-methyl-[1,3,4]oxadiazol-2-yl)-methyl]-7-chloro-1,2,3,4-tetrahydro-cyclopenta[b]indole | 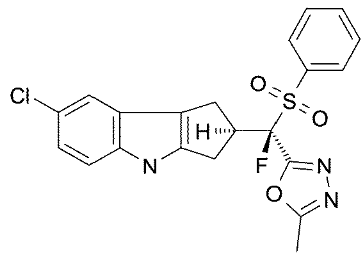 |

The synthesis of Cpd ***C*** (1,1,1,3,3,3-Hexafluoro-2-{2-methyl-1-[5-methyl-2-(3-trifluoromethylphenyl)-oxazol-4-ylmethyl]-1H-indol-5-yl}-propan-2-ol, LXR agonist) was described in WO 2005/105791, Example 56.

Cpd ***D*** (4-{5-[(RS)-(3-Bromo-benzenesulfonyl)-((SR)-7-chloro-1,2,3,4-tetrahydro-cyclopenta[b]indol-2-yl)-fluoro-methyl]-[1,3,4]oxadiazol-2-yl}-benzoic acid partial LXR agonist) was described in WO 2005/092856, Example 113.

Cpd ***E*** ((R)-2-[(S)-Benzenesulfonyl-fluoro-(5-methyl-[1,3,4]oxadiazol-2-yl)-methyl]-7-chloro-1,2,3,4-tetrahydro-cyclopenta[b]indole, partial LXR agonist) was described in WO 2005/092856, Example 74(6).

## Materials and Methods

For in vitro experiments lyophilized compounds were reconstituted in DMSO (Sigma) and diluted in the same solvent to generate 20 mM, 10 mM, 5mM, 1 mM and 0 mM stocks which were stored at -20 °C.

For *in vivo* experiments lyophilized compounds were suspended in vehicle (a specific formulation designed at Roche: 1.25% Hydroxypropyl methyl cellulose, 0.10 % docusate sodium salt, 0.18% propyl paraben sodium, 0.02% citric acid monohydrate, pH 6). A fine particle suspension was ensured by 3 brief pulse sonications on ice. Compound concentration in suspension was adjusted to 2 mg/ml for Cpd C (LXR agonist); and two concentrations, 6 mg/ml and 20 mg/ml, for both ABCA1 inducers, Cpd A and Cpd G. Compound suspensions were stored at -20 °C for long term storage, and at 4 °C if compunds were going to be used within a week. Suspension was thoroughly mixed before administration to ensure homogenous dose delivery.

*Cell culture*

Rat Collagen Type I, RPMI and ITS were purchased from Corning. FBS was purchased from GIBCO and fat-free BSA from Sigma-Aldrich. For cholesterol efflux assay, Human ApoA1 and $^3$H-Cholesterol were purchased from Calbiochem and American Radiolabeled Chemicals, respectively.

Conditionally immortalized human podocytes were a kind gift from Moin Saleem. Cells were seeded in Collagen Type I-coated flasks and propagated at 33 °C, 5 % $CO_2$ in full media (RPMI, 10 % FBS, 1 X Pen/Streptomycin), supplemented with 1 X ITS. For differentiation, cells were seeded at a density of 2,500 cells/cm$^2$ in full media media without ITS and cultured at 37 °C and 5 % $CO_2$ for 15 days.

*Cytotoxicity*

Podocytes were seeded in 96-well plates (Greiner) and allowed to differentiate for 14 days. Cells were then washed with PBS, and incubated with media (RPMI-0.2 % BSA) with or without vehicle and compounds for 18 h at 37 °C, 5 % $CO_2$. Compound concentrations tested were 1 μM, 5 μM, 10 μM and 20 μM. Vehicle (DMSO) final concentration was 0.1 %. All treatments were done in duplicates. Cytotoxicity was assayed using ApoTox-Glo Triplex Assay (Promega) following manufacturer's instructions. Briefly, cell-permeant (GF-AFC) and cell impermeant (bis-AAF-R110) peptide substrates were diluted and added to all wells and the cells were incubated at 37 °C, 5 % $CO_2$ for 1 hr. A set of cells were treated with saponin (2 mg/ml) as a positive control of cell cytotoxicity. Cell viability and cytotoxicity fluorescence signals were measured at 400 nm /505 nm and 485 nm/ 520 nm Excitation/Emission, respectively using a fluorescence microplate reader (SpectraMax i3). RFU signal obtained with the cell toxicity substrate was normalized to the one obtained with the viable substrate in the same well to eliminate the effect of different cell numbers per well.

*Cholesterol Efflux*

Human podocytes differentiated for 13 days were labeled with 1 μCi/ml of [$^3$H]-cholesterol in RPMI media with 2% FBS for 24 h. Cells were then washed with PBS and incubated with equilibration media (RPMI-0.2 % fat free-BSA) supplemented with vehicle or compounds Cpd C (1μM), Cpd A (1 μM, 5 μM) and Cpd G (1 μM, 5 μM, 10 μM) for 18h. Cells were then washed with PBS, and incubated with equilibration media with or without 20 μg/ml of human ApoA1 for 18 h at 37 °C, 5 % $CO_2$. Media was collected, spun at 12,000 x g for 5 min and the radioactivity of the 200 μl aliquot was counted by liquid scintillation. Cells were washed with PBS, lysed with 250 μl 0.1 % SDS, 0.1 M NaOH and the radioactivity in 100 μl aliquot was measured by liquid scintillation. Cholesterol efflux was calculated as the % of labeled cholesterol that moved from the cell to the media by the formula:

$$\text{Efflux (\%)} = 100 \times \{ (\text{cpm media}) / [(\text{cpm media}) + (\text{cpm lysate})] \}$$

ApoA1-mediated cholesterol efflux was calculated as the difference between the efflux in the presence or absence of ApoA1. All treatments were done in duplicate. More than 3 independent experiments were performed.

*ABCA1 expression.*

Podocytes differentiated for 14 days were starved in RPMI-0.2 % FBS for 18 h and then treated with freshly prepared dilutions of compounds in full media for 18 h at 37 °C and 5 % $CO_2$. Compound concentrations of 1 μM, 5 μM and 10 μM were evaluated. Cells were then washed with PBS and lysed with 1 X cell lysis buffer (Cell Signaling) supplemented with protease and phosphatase inhibitor cocktails (Roche). Total protein content was determined by BCA method (Pierce, Thermoscientific). ABCA1 expression was analyzed by western blot.

*Cell fractioning*

14 day-differentiated podocytes were treated with compounds Cpd C (1 μM), Cpd A (5 μM) and Cpd G (10 μM) for 18 h and then washed and scraped with ice cold PBS. Cells were centrifuged at 1,000 x g for 5 min, supernatant was carefully aspirated and the pellet resuspended in hypotonic buffer (15 mM KCl, 1.5 mM $MgCl_2$, 10 mM HEPES and 1mM DTT) supplemented with Roche's protease inhibitor cocktail. Further cell rupture was ensured by frothing in a glass douncer and two freeze and thaw cycles. Sucrose was added, 227 mM final concentration, and and the cells were centrifuged at 1,000 x g for 30 min at 4 °C. The supernatant was then transferred to a new tube and centrifuged at 10,000 x g for 15 min at 4 °C. The pellet, containing the microsomal fraction was collected and the supernatant was transferred to a new tube and centrifuged at 100,000 x g for 1 hr at 4 °C. The pelleted plasma membrane fraction was collected and the supernatant, containing non membranous cytosolic fraction, was concentrated in Vivaspin 500 filter columns. The presence of ABCA1, and proteins known to be present at the plasma membrane ($Na^+/K^+$ sodium pump) and cytosol (MEK), in each fraction was verified by western blot.

*Western blot.*

Lysates, or cell fractions were incubated with buffer sample at 55 °C for 10 min under reducing conditions. A total of 30 ⌠ g of protein in lysates, and one third of the volume collected in cell fraction preps, were separated by SDSPAGE in 4-20 % gels (Biorad) and proteins were then transferred to PVDF membranes. Membranes were blocked with 5 % milk and blotted with protein-specific antibodies for 18 h at 4 °C. The following antibodies were used: mouse anti-ABCA1 (Abeam;

1:1,000), rabbit anti-GAPDH antibody (Millipore; 1:10,000), rabbit anti-MEK and anti-$Na^+/K^+$ ATPase (Cell Signaling; 1:1,000). After washing with PBS-T, membranes were incubated with antimouse and anti-rabbit specific antibodies conjugated to HRP (Promega) 1:10,000 in 5% milk . Membranes were washed and signal was developed using Western bright ECL substrate (Advansta).

*In vivo experiments*

*Study approval*

Study protocols to test the compounds in mice were approved by the University of Miami's IACUC. The University of Miami (UM) has an Animal Welfare Assurance on file with the Office of Laboratory Animal Welfare, NIH (A-3224-01, effective November 24, 2014). Additionally, UM is registered with the US Department of Agriculture Animal and Plant Health Inspection Service, effective December 2014, registration 58-R-007. As of October 22, 2013, the Council on Accreditation of the Association for Assessment and Accreditation of Laboratory Animal Care (AAALAC International) has continued UM's full accreditation.

*Adriamycin-induced kidney damage*

Female Balb/c mice purchased were purchased from Jackson Labs at the age of 6 weeks and housed at our facility for two weeks before starting the experiment.

First, we performed a preliminary experiment to explore the best dose of compounds to be used and to determine if other experimental variables needed to be adjusted. In this experiment, 30 mice received a single dose of Adriamycin (Sigma-Aldrich, 12 mg/Kg, via tail vein injection) and then were randomly separated into 6 groups of 5 mice each. Another 5 mice were injected with 0.9 % NaCl by the same route and were used as a no-kidney injury baseline group. Starting 24 h after ADR injection, vehicle, or different doses of compounds, were given once daily by oral gavage for 35 days. The doses of compounds tested were the following: LXR agonist (Cpd C, 10 mg/Kg); ABCA1 inducers Cpd A and Cpd G (30 mg/Kg and 100 mg/Kg). Body weight measurements and spot urine collection was done once a week. Animals were sacrificed 35 days after ADR injection (Fig 1).

A second experiment was repeated to confirm the improvement of renal function attained with the doses of Cpd A and G in the first experiment. Briefly, 30 female Balb/c mice of 8 weeks age were injected with 12 mg/Kg of ADR by via tail vein and the mice were randomly distributed into 5 groups of 6 animals each. Animals were given vehicle or compounds by oral gavage once daily for 28 days starting one day after ADR injection. The following doses of compounds were tested: Cpd C, 10 mg/Kg/day; Cpd A, 30 mg/Kg/day and Cpd G, 30 mg/Kg/day and 100 mg/Kg/day. A group of 5 animals that received saline solution via tail vein injection and were treated with vehicle were used as healthy baseline control. Body weight measurements and spot urine collection was done once a week. Animals were sacrificed 28 days after ADR injection (Fig.2).

*Alport mouse model*

129 Col4a3$^{tm1/Dec}$/J mice were purchased from Jackson Labs and bred to generate Col4a3 KO mice. Mice were genotyped and selected using the primers: F (TGCTCTCTCAAATGCACCAG), R (CCAGGCTTAAAGGGAAATCC) and Rm (GCTATCAGGACATAGCGTTGG) in a PCR reaction (94C for 5 min, followed by 30 cycles of 30 sec at 95C, 15 sec at 60C and 30 sec 72C, and 1min at 72C). 129 Col4A3-KO mice were divided into two groups of 5 mice (males and females) each. At the age of 4 weeks, mice started receiving either vehicle or Cpd G (100 mg/Kg) by oral gavage once daily for 4 weeks. Body weight measurements and spot urine collection was performed once a week. Animals were sacrificed at the end of the treatment at the age of 8 weeks. A late study was also performed to evaluate if tretament of Col4A3-KO mice at time of established disease would prolong mice survival. In this study, mice were treated with Cpd G (100 mg/Kg/day) by daily oral gavage starting at 6 weeks of age and survival was assessed.

*DKD mouse model*

B6.BKS$^{db/db}$ and B6.BKS$^{db/+}$ mice were purchased from the Jackson laboratory. At the age of 14 weeks, mice started receiving vehicle or the ABCA1 inducer Cpd A (30 mg/Kg) by oral gavage once daily for 4 weeks. Body weight measurements and urine collection was performed every week. Mice were sacrificed at 18 weeks old, blood was collected and tissues were processed and analyzed as described below.

*Phenotypic analysis of mice*

Spot urine samples were collected for all mice at baseline and time of sacrifice. Urinary albumin and creatinine were determined using an albumin ELISA kit (Bethyl laboratories) and a colorimetric assay for creatinine determination (Stanbio). Albuminuria was calculated and expressed as μg of albumin divided by mg of creatinine. For diabetic mouse model, weight and glycaemia were measured on a biweekly basis.

At time of sacrifice blood was collected in heparinized tubes and mice were perfused via left ventricle perfusion with isotonic saline solution. Kidney cortex was carefully excised and further sectioned for the fallowing analysis : determination of podocyte number, lipid droplet staining, electron microscopy (EM), renal lipids content assays, PAS& HE staining for pathological evaluation.

*Determination of podocyte number*

Kidney cortex sections were embedded in OCT for further analysis of immunofluorescent staining and lipid droplet. Specifically, for determination of podocyte number per glomerular cross-section was determined using 4 μm-thick tissue sections that were cut and stained with WT1 antibody (1:200, Santa Cruz) and prolong GOLD DAPI mounting media. Images were acquired with confocal microscopy using a Leica SP5 inverted microscope with the 40x wet objective. 20 glomeruli per mouse were quantified.

Lipid droplet staining Filtered Oil-Red O-Isopropanol solution (Electron Microscopy Science, PA) was diluted with water (6:4). 4 μm kidney sections were incubated with 100μl freshly prepared Oil-Red O solution (ORO) for 15 minutes and counterstained with Hematoxylin Harris Hg Free (VWR, PA) to detect lipid deposition. Glomeruli staining was evaluated using a light microscope (Olympus BX 41, Tokyo, Japan).

Electron microscopy. For transmission electron microscopy kidney cortex sections were placed in 4% paraformaldehyde, 1% gluteraldehyde in 0.1 M phosphate buffer (pH 7.4). Foot processes were quantified per 1 μm of the glomerular basement membrane.

Renal lipids species assays. Kidney cortex sections were snap frozen and utilized for lipid extraction and cholesterol content determination. The tissue was homogenized in 2 mM potassium phosphate buffer in a glass douncer on ice. Lipids in 100 μl homogenate (~5-10 mg tissue) were extracted with 1 ml hexane: isopropanol (3:2) for two sequential 30-minute extractions. The solvent containing lipids was then dried in Nitrogen atmosphere. Lipids were then reconstituted using isopropanol: NP-40 (9:1) and cholesterol content was quantified using the Amplex red cholesterol assay kit (Invitrogen) according to the manufacturers protocol. Triglycerides in lipid extracts were assayed with a colorimetric kit according to manufacturer's instructions (Cayman). Total cholesterol and cholesterol esters were assayed using an enzymatic fluorometric method. For total cholesterol renal lipid extracts were diluted in assay buffer (100 mM potassium phosphate, 50 mM NaCl, 5 mM cholic acid, 0.1% Triton X-10, pH 7.4) and incubated with same buffer supplemented with a final concentration of 1 U/ml cholesterol oxidase, 1 U/ml cholesterol esterase, 1 U/ml horseradish peroxidase, and 75 μM Amplex Red. The reactions were incubated at 37 °C for 30 min in a black opaque 96 well plate (Greiner) and fluorescence was measured in a microplate reader (Spectramax i3X, Molecular Devices) using 530 nm excitation and 580 emission.

Cholesterol esters were assayed using a direct method described by Mizoguchi et al (Mizoguchi, 2004). Briefly 150 μl of FC decomposition reagent (45 U/ml bovine catalase and 1 U/ml cholesterol oxidase, in assay buffer described above) was added to 25 μl of sample containing up to 1 mM total cholesterol. Free cholesterol was allowed to decompose overnight at 37 °C and then 75 μl of 4X cholesterol ester detection reagent (1 U/ml cholesterol oxidase, 4 U/ml cholesterol esterase, 24 U/ml horseradish peroxidase, 300 μM Amplex Red) was added. Reaction was incubated at 37 °C for 30 min and fluorescence was measured as described above for total cholesterol. 1 mM of cholesterol and 5 μM of cholesterol oleate standards were included as internal controls to validate assay sensibility and specificity.

Pathologocical evaluation. Kidney cortex sections were paraffin-embedded and cut at 4μm thick for periodic acid-Schiff (PAS) and HE . Mesangial expansion was scored based on semi-quantitative analysis (scale 0-5) or percent of glomeruli with mesangial expansion (%), performed in a double-blind manor.

*Statistics*

Statistics. All values are represented as means with SD. Statistical analysis was performed using Prism GraphPad 7 software. Results were analyzed using 1-way ANOVA if more than one compound dose was used and goups showed normal data distribution. If differences were observed, the means of the control (vehicle) was compared to mean of each group using Dunnett's test. Tukey test was performed for multiple comparisons.

For in vivo studies, vehicle and Cpd-treated groups were compared using double-tailed t-test. A Welch's correction was used in case of unequal variances. Groups were also compared using a Mann-Whitney test when normality distribution could not be asserted. P values < 0.05 % were considered statistically significant.

## Results.

### Compound cytotoxicity.

We performed cell cytotoxicity assays to determine the Cpd concentration range that could be safely used in cultured human podocytes.

All compounds can be used at concentrations up to 10 μM without signs of cytotoxicity. However, we decided not to exceed a concentration of 5 μM when using Cpds A and C, as these two compounds induced significant toxicity at 20 μM (Fig. 3)

### ABCA1 expression and cholesterol efflux

We performed in vitro experiments to select the compounds that were better at inducing functional expression of ABCA1 in podocytes for further use in animal studies. Thus, we studied the effect of compounds on ABCA1 protein expression, ABCA1 localization at the plasma mebrane and ApoA1-mediated cholesterol efflux.

ABCA1 expression induced by compounds was addressed by western blot. All LXR agonists, Cpd C, Cpd E and Cpd D, markedly increased ABCA1 expression in podocytes at doses as low as 1 uM. Of the three ABCA1 inducers tested (Cpd A, Cpd F and Cpd G), only Cpd A, and Cpd G, increased ABCA1 expression, but at 10 uM, and not as markedly as LXR agonists (Fig 4a).

To address whether the increased ABCA1 expression observed was associated with the production of more functional protein, we performed cell fractionation of podocytes treated with 1 μM Cpd C, 5 μM Cpd A and 10 μM Cpd G. The increased expression of ABCA1 with the drug concentration used was first verified (Figure 4b) and ABCA1 localization in each of the fractions obtained was checked by western blot. Fractions obtained were also blotted for protein known to be located on the plasma membrane and the cytosolic fractions, $Na^+/K^+$ ATPase and MEK, respectively, to verify successful cell fractionation (Fig 4c-e). The doses of the three compounds used promoted ABCA1 localization at the plasma membrane (Fig 4c) and, as expected, no ABCA1 was found in the nonmembrane cytosolic fraction. Less ABCA1 was found in the microsomal compartment of cells treated with ABCA1 inducers Cpd A and Cpd G (Fig 4 d), suggesting that these compounds are more effective at promoting localization of this protein at the plasma membrane than LXR agonists. In addition, we performed cholesterol efflux assays using ApoA1 as cholesterol carrier. This assay measures functionality of ABCA1 in regard to cholesterol efflux as ABCA1 specifically interacts with ApoA1 for the transfer of cholesterol from cells to nascent HDL particles. Significant increase of ApoA1-mediated efflux could be achieved with 1 μM Cpd C (LXR agonist), 5 μM Cpd A and 10 μM Cpd G (Figure 4 d-e).

Based on these in vitro experiments we chose ABCA1 inducers Cpd A and Cpd G to test in an animal model of kidney injury, along with LXR agonist Cpd C, as a reference.

### ABCA1 inducers protects from experimental FSGS

The ADR model of kidney injury is a drug induced model of proteinuric kidney disease and remains the most widely used experimental model of focal segmental glomerulosclerosis (FSGS). A dose range finding experiment was performed with compounds selected from in vitro experiments.

ADR injection induced severe transient proteinuria and body weight loss. Albuminuria and body weight were checked weekly. No significant differences were observed in the group treated with LXR agonist (Cpd C, Figure 5a). However, proteinuria was reduced in the groups that received 30 mg/Kg of Cpd A and 100 mg/Kg of Cpd G (Figure 5,b and e), and, to a lesser extent, in the group that received 30 mg/Kg of Cpd G (Figure 5d). Similarly, animals injected with ADR experienced a 10-20 % body weight loss which was prevented in animals treated with 30 mg/Kg of Cpd A and 100 mg/Kg of Cpd G (Fig 5,g and j) but not with LXR agonist (Fig 5,f).

In a second independent in vivo experiment, we utilized a larger number of mice and selected the doses of Cpd A and G found to be beneficial in prior experiment, i.e. 30 mg/Kg of Cpd A and 100 mg/Kg of Cpd G. Similarly as in previous experiment, both ABCA1 inducers reduced albuminuria and body weight loss (Figure 6b).

As compound G was found to be the one with the best beneficial effect, and the ADR model of kidney disease does not present with impaired renal function, we perform additional quantitative histological studies in animals that were treated with this compound. A blind pathological study of kidney cortex revealed a reduction of global and segmental glomerular sclerosis, podocyte hypertrophy and hyperplasia, tubular microcysts and interstitial inflammation in animals that received 100 mg/Kg/day of Cpd G (Figure 7). These data together demonstrated that compound G was highly effective in reducing hallmarks of FSGS caused by Adriamycin and was more effective than an LXR agonist assessed as a comparator.

As we were administering compounds that were expected to influence cholesterol metabolism, we measured cholesterol and triglyceride levels in the blood of the experimental animals. No significant differences could be found for

both clinical parameters (Tables 1 and 2).

**Table 1.** Blood cholesterol and triglycerides in animals treated with vehicle, LXR agonist (Cpd C) and ABCA1 inducers (Cpd A and Cpd G) for 35 days after ADR injection.

| | saline VEHICLE - | **adriamycln VEHICLE** - | **adriamycin Opd C(RO4897308)** 10 mg/Kg | **adriamycin Opd A (RO5486083)** 30 mg/Kg | **adriamytin** 100 mg/Kg | **adriamycin Opd G (RO52676836)** 30 mg/Kg | **adriamycin** 100 mg/Kg |
|---|---|---|---|---|---|---|---|
| **TOTAL CHOL [mg/dL]** | 96.6 ± 15.6 | 153.3 ± 64.8 | 117.4 ± 66.8 | 86.3 ± 8.8 | 226.7 ± 170.4 | 136.8 ± 38.9 | 109.7 ± 11.0 |
| **TRIGLYCERIDES [mg/mL]** | 85.4t31.7 | 51.8 ± 14.5 | 57.2 t8.3 | 57.8 ±15.5 | 47.3 ± 7.5 | 51.0 ±16.8 | 82.0 t15.5 |

**Table 2.** Blood cholesterol and triglycerides in animals treated with vehicle, LXR agonist (Cpd C) and ABCA1 inducers (Cpd A and Cpd G) for 28 days after ADR injection.

| | saline VEHICLE - | **adriamycin** VEHICLE - | **adriamycin** **Opd C(RO4897308)** 10 mg/Kg | **adriamycin** **Opd A(RO5486083)** 30 mg/Kg | **adriamycin** **Opd G(RO5267683)** 30 mg/Kg | **adrismycin** 100 mg/Kg |
|---|---|---|---|---|---|---|
| **TOTAL CHOL [mg/dL]** | 87.4 ± 7.1 | 244.6 ± 207.4 | 119.8 ± 19.9 | 104.0 ± 31.6 | 112.0 ± 13.6 | 101.1 ± 12.8 |
| **TRGLYCERIDES [mg/mL]** | 117.2 ± 19.1 | 130.5 ± 21.1 | 110.0 ± 53.5 | 107.2 t 23.9 | 119.8 t 18.8 | 121.0 ±19.3 |

Lipid deposition in renal tissue has been documented in the setting of kidney injury. We performed Oil Red O (ORO) staining to determine if there was lipid accumulation in animals injected with ADR and whether Cpd G could reduce this effect. Significant deposition of lipid droplets was found in the kidneys of ADR-injected mice, which was significantly reduced in animals treated with Cpd G (Figure 8). Indeed, ADR-injected mice that had received vehicle showed significant lipid deposition in kidney cortexes and in contrast, Cpd G completely prevented the accumulation of glomerular lipids in response to ADR demonstrating no detectable accumulation of lipids in kidney cortexes in comparison to age-matched normal control mice.

To determine the species of lipids accumulated in the renal tissue we extracted the lipids for kidney cortex and analyzed them for triglycerides, total cholesterol and cholesterol esters. We could not find differences in the content of triglycerides and total cholesterol between kidneys from animals injected with ADR or plain saline solution. However, we found a significantly higher content of esterified cholesterol in ADR-injected mice, which was significantly reduced by Cpd G treatment (Figure 9a-c). There was a strong correlation between the severity of albuminuria and the cholesterol esters content detected in renal tissue (Figure 9d) but not with the other lipid species analyzed (Figure 9e and f). Cpd G treatment of ADR-injected mice prevented accumulation of cholesterol esters and without wanting to be bound by any theory it is believed that this compound may improve glomerular cholesterol removal via an upregulation of ABCA1 .

We next investigated compounds C, A and G toxicity by serology. More specifically, we determined hematocrit, hemoglobin white cell blood count and liver transaminases ALT and AST of all treated animals to determine if liver toxicity and blood dyscracias were associated with the compounds. No apparent signs of such toxicities were found in experimental mice (Table 3)

**Table 3:** Animals treated with compounds C, A or G did not shown abnormalities in WBC, hemoglobin, hematocrit and the levels of ALT and AST transaminases after a 35-day treatment with compounds when compared to vehicle treated controls. Data represent the mean and standard deviation of each parameter.

| | saline VEHICLE - | **adriamycin** **VEHICLE** - | **adriamycin** **RO4897308 (C)** 10 mg/Kg | **adriamycin** **RO5486083 (A)** 30 mg/Kg | **adriamycin** **RO5267683 (G)** 30 mg/Kg | **adriamycin** 100 mg/Kg |
|---|---|---|---|---|---|---|
| **WBC [x10^3 cellas/ul]** | 6.3 ± 2.2 | 9.7 ± 3.7 | 7.6 ± 4.4 | 4.6 ± 1.3 | 6.3 ± 1.2 | 4.7 ± 1.6 |
| **HEMOGLOBIN [g/dL]** | 13.2 ± 0.7 | 11.1 ± 3.2 | 12.2 ± 1.0 | 13.9 ± 1.1 | 12.9 ± 1.0 | 13.1 ± 0.6 |
| **HCT/[%]** | 49.0 ± 3.8 | 43.3 ± 12.2 | 44.8 ± 1.5 | 51.8 ± 3.3 | 49.5 ± 4.4 | 49.6 ± 2.3 |
| **ALT[U/L]** | 74.0 ± 49.1 | 397.5 ± 221.1 | 299.5 ± 365.0 | 339.3 ± 274.8 | 181.6 ± 69.0 | 191.4 ± 79.9 |
| **ASR[U/L]** | 119.4 ± 32.6 | 376.0 t 104.4 | 213.0 ± 151.7 | 365.3 ± 181.2 | 323.4 ± 87.1 | 212.4 ± 91.1 |

Animals treated with optimal dose of Cpd G had better physical appearance than those that received ADR throughout the experiment. This was particularly noticeable during the 3rd and 4 th week of treatment (Figure 10).

**Cpd G protects from kidney failure in mouse model of progressive kidney disease (Alport Syndrome)**

Untreated CoL4a3 KO mice develop severe albuminuria and high BUN and serum creatinine levels between weeks 4 and 8 of age. These mice reach ESRD by 8 weeks of age and do not survive beyond that time point.

To study the nephroprotective effect of Cpd G in this model of Alport Syndrome, we treated CoL4a3 KO mice for 4 weeks with 100 mg/Kg of Cpd G or with vehicle starting at the the age of 4 weeks. Animals treated with 100 mg/Kg of Cpd G showed a delayed progression to ESRD, with significantly less albuminuria, BUN and serum creatinine compared to animals that just received vehicle (see Figure 11). Animals treated with Cpd G also lost less weight than those that received vehicle.

Histological analysis of kidney sections revealed significantly less mesangial expansion in Cpd G treated Col4a3 KO mice than in vehicle treated Col4a3 KO. A separate study was conducted to investigate if treatment with Cpd G in mice with established kidney failure would result in improved survival. Indeed, even when the treatment with Cpd G was started in 6 weeks old mice with relatively advanced CKD, the improvement in kidney function in Cpd G treated Col4a3 KO mice was associated with reduced mortality resulting in an increased life span of almost 15%.

Finally, kidney cortex sections from the 8 week old Col4a3 KO mice showed significant lipid accumulation (increased Oil-red O staining), which was reduced in animals that received Cpd G (see Figure 13). Consistent with the previous results in the ADR model, cholesterol ester in kidneys of Col4a3 KO mice, which was significantly higher than WT littermates, was significantly reduced by the treatment with Cpd G.

**CpdA partially protects from a mouse model of diabetic kidney disease Increased ABCA1 reverts podocyte injury and DKD**

We aimed to validate ABCA1 as a therapeutic target for DKD in the obese diabetic db/db mouse model. Db/db mice treated with a pharmacological inducer of ABCA1 experienced a reduction in albuminuria after two weeks of treatment (16 weeks) and an even further reduction after four weeks of treatment (18 weeks) (Figure 12A) compared to db/db vehicle treated mice. Blood urea nitrogen (BUN) was found to be significantly improved (Figure 12B), which correlated with a significant reduction in cholesterol esters (Fig 12C-D) in the ABCA1 inducer (compound A) treatment group as compared to the db/db mice. Kidney cortex sections were used for various histological assessments that showed that ABCA1 inducer treatment in db/db mice experience improved: podocyte number as determined by quantifying WT1 positive cells (Fig 12E-F), mesangial expansion as determined using PAS stained sections (Fig 12G-H), and podocyte foot process effacement as determined using TEM images (Fig 12I-J).

**Claims**

1. **A** compound for use in treating kidney diseases, wherein said compound is an ABCA1 inducer compound.

2. The compound for use according to Claim 1, **characterized in that** it is represented by the formula I

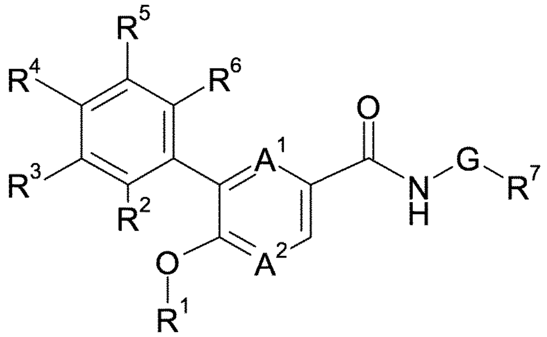

**I**

wherein

one of $A^1$ and $A^2$ is N and the other one of $A^1$ and $A^2$ is CH;
$R^1$ is selected from the group consisting of $C_{1-7}$-alkyl, $C_{3-7}$-cycloalkyl, $C_{3-7}$-cycloalkyl-$C_{1-7}$-alkyl, $C_{1-7}$-alkoxy-$C_{1-7}$-alkyl, halogen-$C_{1-7}$-alkyl, heterocyclyl-$C_{1-7}$-alkyl wherein the heterocyclyl group is unsubstituted or substituted by oxo, and heteroaryl-$C_{1-7}$-alkyl wherein the heteroaryl group is unsubstituted or mono- or di-substituted by lower alkyl;

$R^2$ and $R^6$ independently from each other are hydrogen or halogen;
$R^3$ and $R^5$ independently from each other are selected from the group consisting of hydrogen, $C_{1-7}$-alkyl, $C_{1-7}$-alkoxy, halogen, halogen-$C_{1-7}$-alkyl, halogen-$C_{1-7}$-alkoxy and cyano;
$R^4$ is selected from the group consisting of hydrogen, $C_{1-7}$-alkyl, $C_{1-7}$-alkoxy, halogen, halogen-$C_{1-7}$-alkyl, halogen-$C_{1-7}$-alkoxy, amino and cyano;
$R^7$ is selected from the group consisting of $C_{1-7}$-alkyl, $C_{3-7}$-cycloalkyl, said cycloalkyl being unsubstituted or substituted by hydroxy, heterocyclyl, said heterocyclyl having 3 to 7 ring atoms, comprising one, two or three heteroatoms selected from N, O and S and being unsubstituted or substituted by hydroxy or oxo, phenyl, wherein phenyl is unsubstituted or substituted by one or two groups selected from the group consisting of $C_{1-7}$-alkyl, hydroxy, $C_{1-7}$-alkoxy, cyano, halogen and halogen-$C_{1-7}$-alkyl, and heteroaryl, wherein heteroaryl is unsubstituted or substituted by one or two groups selected from the group consisting of $C_{1-7}$-alkyl, hydroxy, $C_{1-7}$-alkoxy, cyano, halogen and halogen-$C_{1-7}$-alkyl, and
G is selected from the group consisting of $-(CH_2)m-$, wherein m is selected from 0 or 1, and $-NR_8-$, wherein $R_8$ is hydrogen or $C_{1-7}$-alkyl,
and pharmaceutically acceptable salts thereof.

**3.** The compound for use according to claim 1 or 2, wherein G is a bond and $R_7$ is $C_{3-7}$-cycloalkyl, said cycloalkyl being unsubstituted or substituted by hydroxy.

**4.** The compound for use according to Claim 3, wherein $R_7$ is cyclohexyl substituted by hydroxy.

**5.** The compound for use according to any one of claims 1 to 4, wherein $R^2$ and $R^6$ are each a hydrogen, $R^4$ is halogen, and one of $R^3$ and $R^5$ is halogen and the other one of $R^3$ and $R^5$ is hydrogen.

**6.** The compound for use according to any one of claims 1 to 5, wherein $R^1$ is halogen-$C_{1-7}$-alkyl.

**7.** The compound for use according to Claim 6, wherein R1 is selected from $-CF_3$, $- CHF_2$, $-CH_2Cl$, $-CH_2CF_3$, $-CH(CF_3)_2$, $-CF_2-CF_3$.

**8.** The compound for use according to any one of claims 1 to 7, which is 6-(3,4-dichlorophenyl)-N-[(1R,2R)-2-hydroxycyclohexyl]-5-(2,2,2-trifluoroethoxy)pyridine-2-carboxamide.

**9.** The compound for use according to any one of claims 1 to 8, which is 5-(3,4-dichloro-phenyl)-N-((1R,2R)-2-hydroxycyclohexyl)-6-(2,2,2-trifluoro-ethoxy)-nicotinamide.

**10.** The compound for use according to any one of claims 1 to 9, wherein said kidney disease is selected from chronic kidney diseases, primary or secondary glomerular diseases or proteinuric kidney diseases.

**11.** The compound for use according to Claim 10, wherein said glomerular disease is Alport syndrome or focal segmental glomerulosclerosis.

**12.** The compound for use according to Claim 10, wherein said kidney disease is selected from diabetic kidney disease.

**13.** The compound for use according to any one of claims 1 to 12, which is formulated for oral administration.

**14.** The compound for use according to any one of claims 1 to 12, which is formulated for topical, intranasal, intraocular, intravenous, intramuscular, subcutaneous, intravitreal, intrathecal or transdermal administration.

**15.** The compound for use according to one of claims 1 to 12, wherein the daily dose is 20 to 800 mg/day.

**16.** The compound for use according to one of claims 1 to 12, wherein the daily dose is 200 mg/day.

**17.** The compound for use according to any preceding claims, wherein the dosing regimen is once daily, twice daily, three time a day, once per three days, once weekly, once every two weeks, or once monthly.

**18.** The compound for use according to any preceding claims, wherein the loading dose regimen double the dose for the first 7 days, 14 days, or 30 days.

**19.** The compound for use according to any one of claims 1 to 16, for the simulatenous, sequential or separate use with a compound selected from the group consisting of angiotensin-converting enzyme (ACE) inhibitor drugs; RAS blockers; angiotensin receptor blockers (ARBs); protein kinase C (PKC) inhibitors; inhibitors of AGE-dependent pathways; anti-inflammatory agents; GAGs; pyridoxamine; endothelin antagonists, COX-2 inhibitors, PPAR-$\gamma$ antagonists and other compounds like amifostine, captopril, cyclophosphamide, sodium thiosulfate, tranilast or cyclodextrins and their derivatives, vitamin D derivatives, anti-hyperglycemic agents and anti-hypercholesterolemic agents.

**20.** A compound of formula I as defined in precedings claims 2 to 9 or a pharmaceutically acceptable salts, for the preparation of a medicament for the treatment and/or prophylaxis of kidney diseases.

**21.** A pharmaceutical composition for use according to any preceding claims, comprising a comppund as defined in claims 1 to 16 and a pharmaceutically acceptable carrier and/or adjuvant.

**22.** A method for treating kidney diseases in a subject in need thereof, comprising administering a therapeutically efficient amount of an ABCA1 inducer as defined in any one of claims 1 to 9.

**23.** The method according to claim 22, wherein said ABCA1 inducer is administrated by topical, oral, intranasal, intraocular, intravenous, intramuscular, subcutaneous, intravitreal, intrathecal or transdermal route.

**24.** The method according to any one of claim 22 or 23, wherein the administration is once daily, twice daily, three time a day, once every three days, once weekly, once every two weeks, or once monthly, preferably one daily.

**25.** The method according to any one of claim 22 or 23, wherein the daily dose of ABCA1 inducer as defined in any one of claims 1 to 9, is in the range of 20 to 800 mg/day.

**26.** The method according to any one of claims 22 to 25, wherein said ABCA1 inducer is administered simultaneously, separately or sequentially in combination with a therapeutically effective amount of an angiotensin-converting enzyme inhibitor or an angiotensin-receptor blocker.

**27.** The method according to any one of claims 22 to 25, wherein said ABCA1 inducer is administered simultaneously, separately or sequentially in combination with a therapeutically effective amount of a compound selected from the group consisting of angiotensin-converting enzyme (ACE) inhibitor drugs; RAS blockers; angiotensin receptor blockers (ARBs); protein kinase C (PKC) inhibitors; inhibitors of AGE-dependent pathways; anti-inflammatory agents, GAGs; pyridoxamine; endothelin antagonists, COX-2 inhibitors, PPAR-$\gamma$ antagonists and other compounds like amifostine, captopril cyclophosphamide, sodium thiosulfate, tranilast, or cyclodextrins and their derivatives, vitamin D derivatives, anti-hyperglycemic agents and anti-hypercholesterolemic agents.

**FIGURE 1**

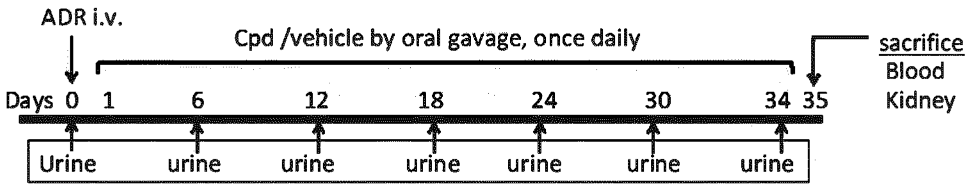


| group | n | Day 0 (i.v. injection, single dose) | Days 1-35 (Cpd treatment) |
|---|---|---|---|
| baseline | 5 | NaCl 0.9 % | Vehicle 5 ml/Kg, oral gavage, once every day |
| vehicle | 5 | ADR 12 mg/Kg | Vehicle 5 ml/Kg, oral gavage, once every day |
| Cpd C (LXR) | 5 | ADR 12 mg/Kg | 10 mg/Kg, oral gavage Once every day |
| Cpd A_30 | 5 | ADR 12 mg/Kg | 30 mg/Kg, oral gavage Once every day |
| Cpd A_100 | 5 | ADR 12 mg/Kg | 100 mg/Kg, oral gavage Once every day |
| Cpd G_30 | 5 | ADR 12 mg/Kg | 30 mg/Kg, oral gavage Once every day |
| Cpd G_100 | 5 | ADR 12 mg/Kg | 100 mg/Kg, oral gavage Once every day |

**FIGURE 2**

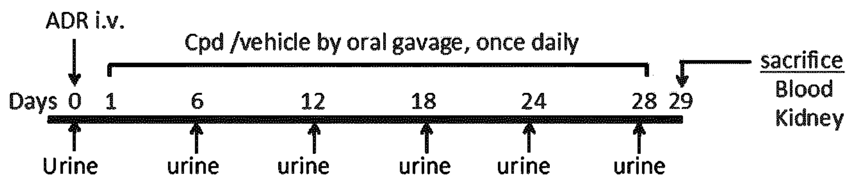


| group | n | Day 0 (i.v. injection, single dose) | Days 1-28 (Cpd treatment) |
|---|---|---|---|
| baseline | 5 | NaCl 0.9 % | Vehicle 5 ml/Kg, oral gavage, once every day |
| vehicle | 6 | ADR 12 mg/Kg | Vehicle 5 ml/Kg, oral gavage, once every day |
| Cpd C (LXR) | 6 | ADR 12 mg/Kg | 10 mg/Kg, oral gavage Once every day |
| Cpd A_30 | 6 | ADR 12 mg/Kg | 30 mg/Kg, oral gavage Once every day |
| Cpd G_30 | 6 | ADR 12 mg/Kg | 30 mg/Kg, oral gavage Once every day |
| Cpd G_100 | 6 | ADR 12 mg/Kg | 100 mg/Kg, oral gavage Once every day |

FIGURE 3

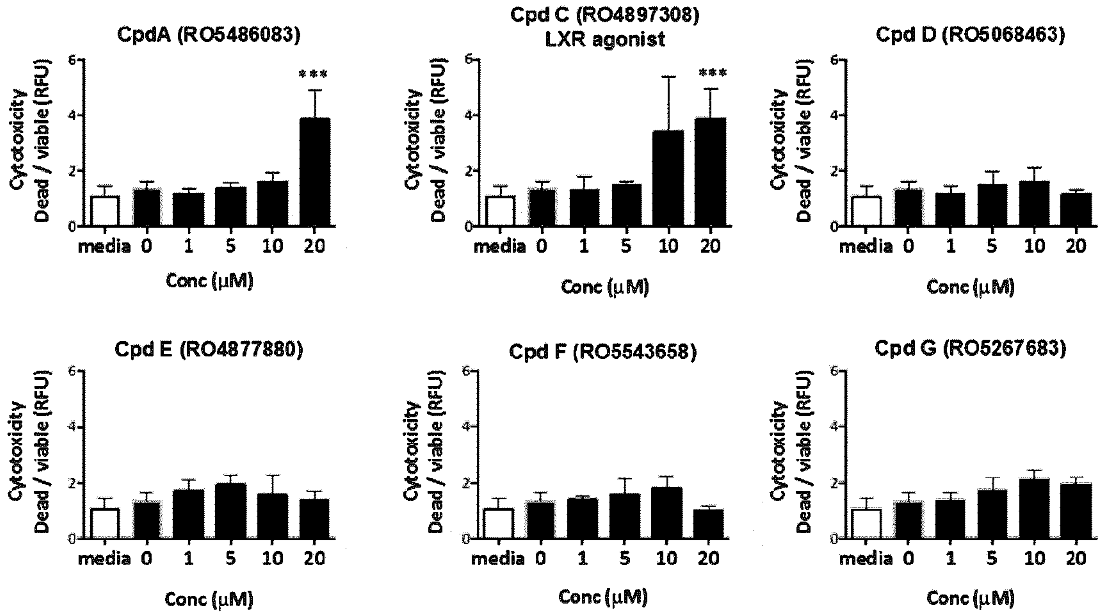


FIGURE 4

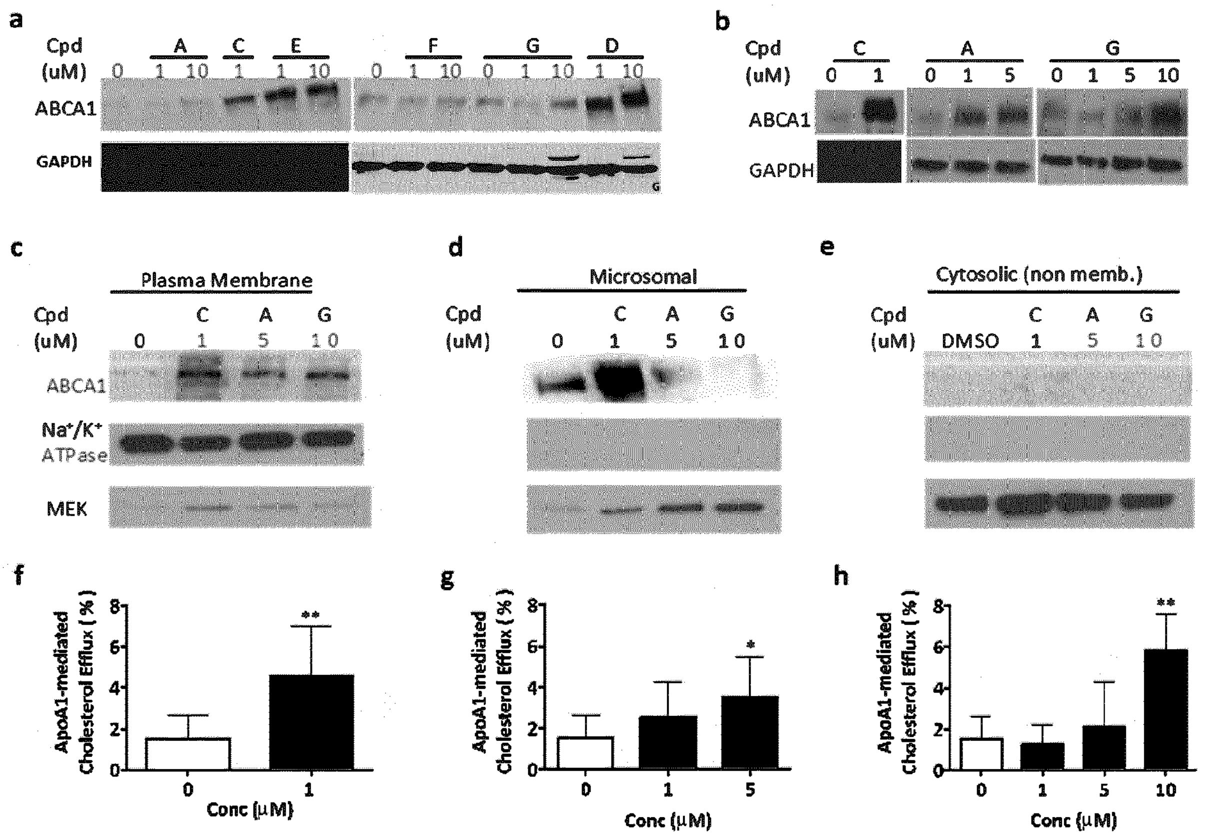

FIGURE 5 (a-e)

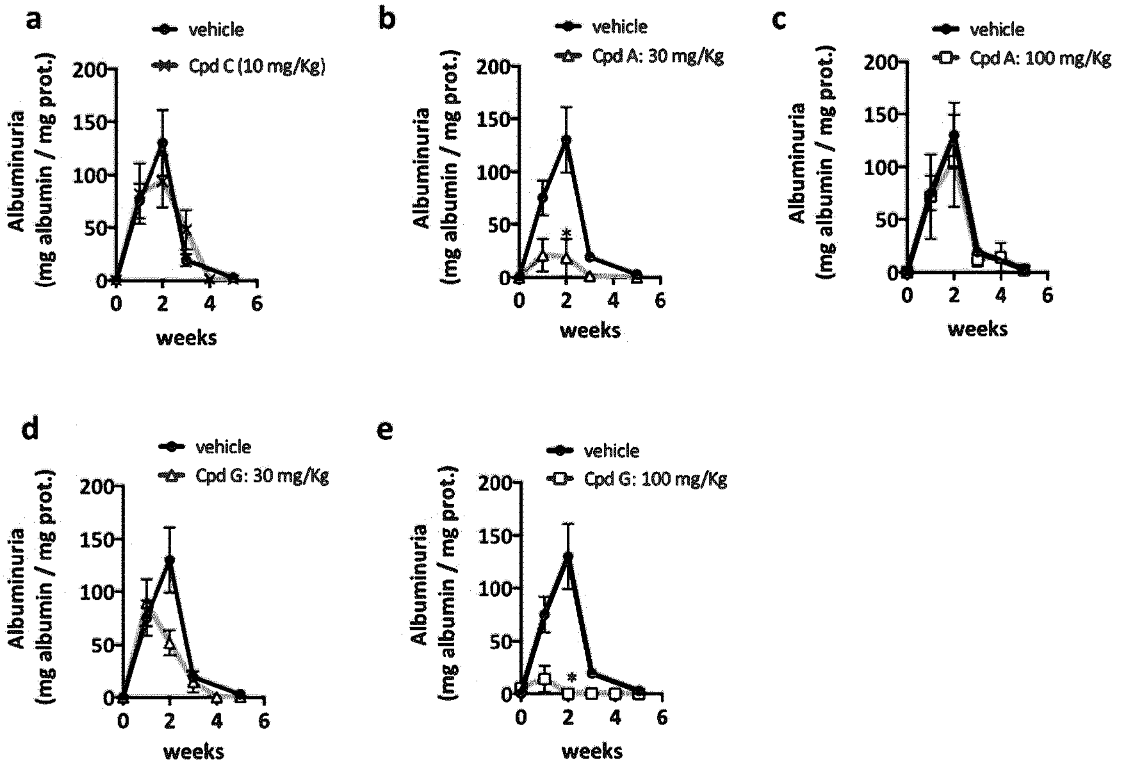


FIGURE 5 (f-j)

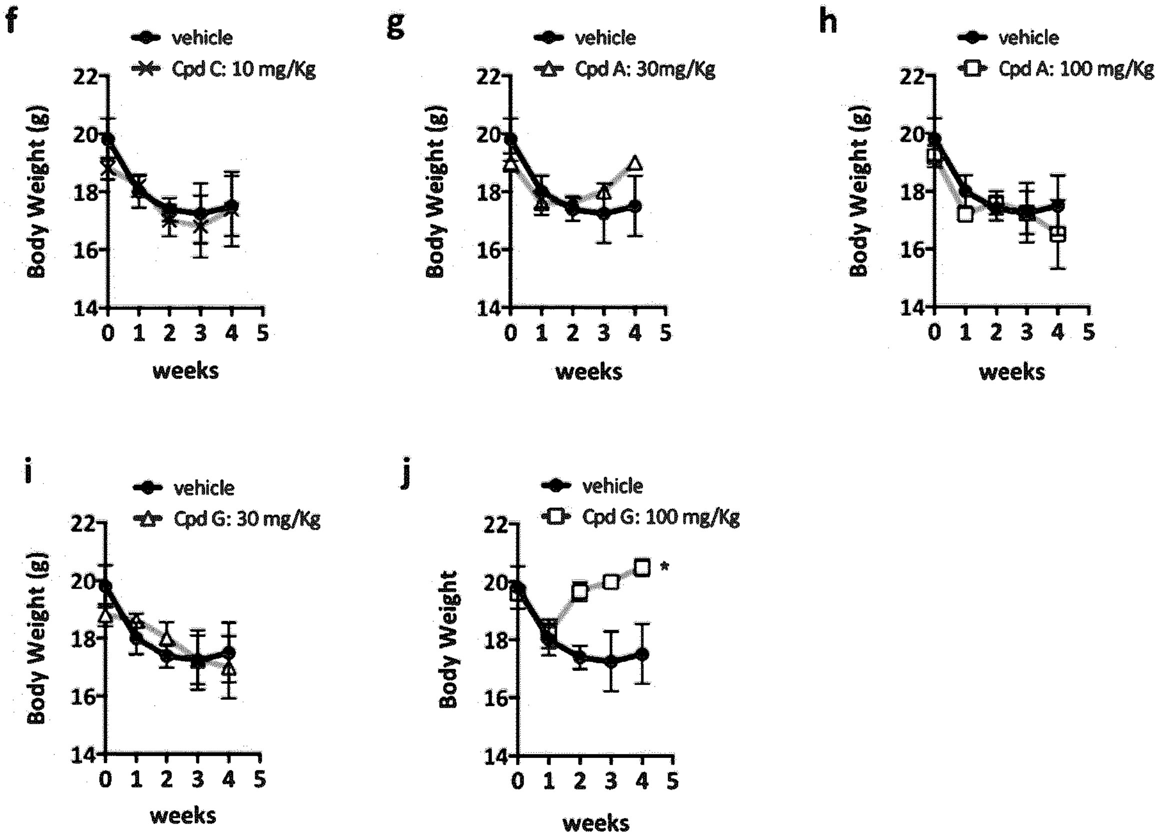

**FIGURE 6**

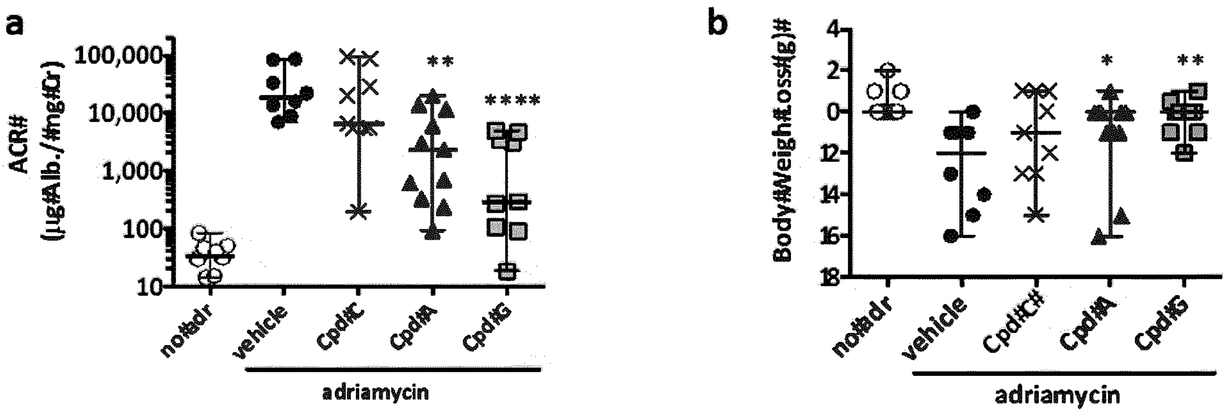


**FIGURE 7**

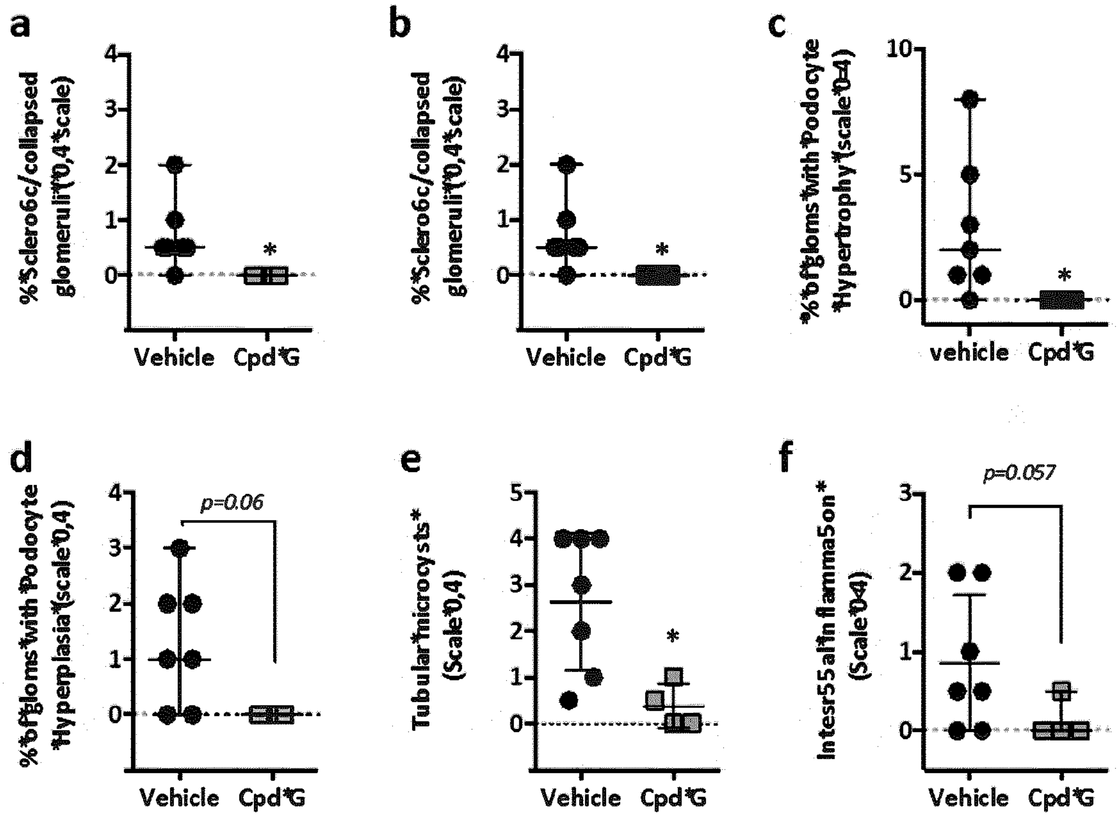

FIGURE 8

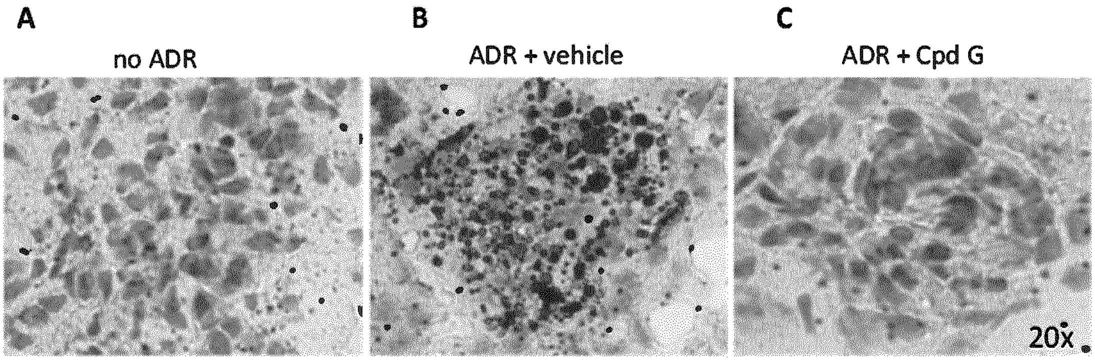


FIGURE 9

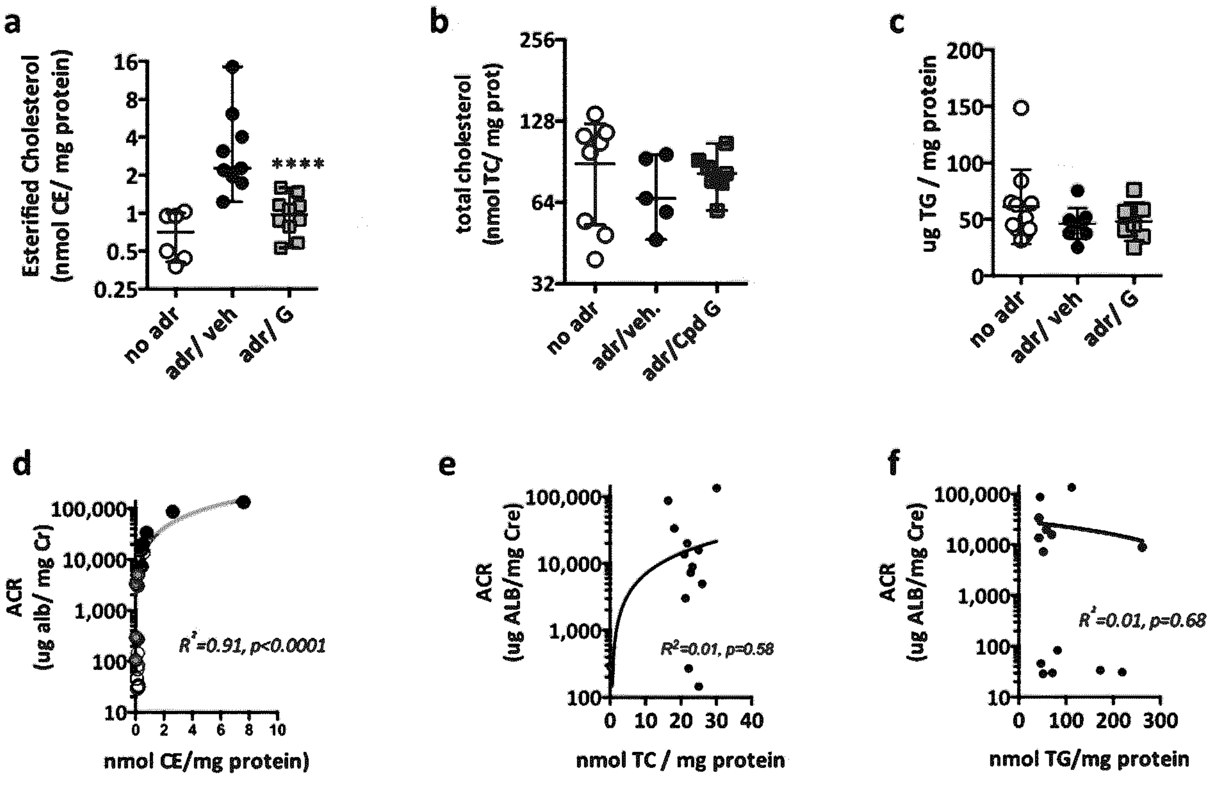

FIGURE 10

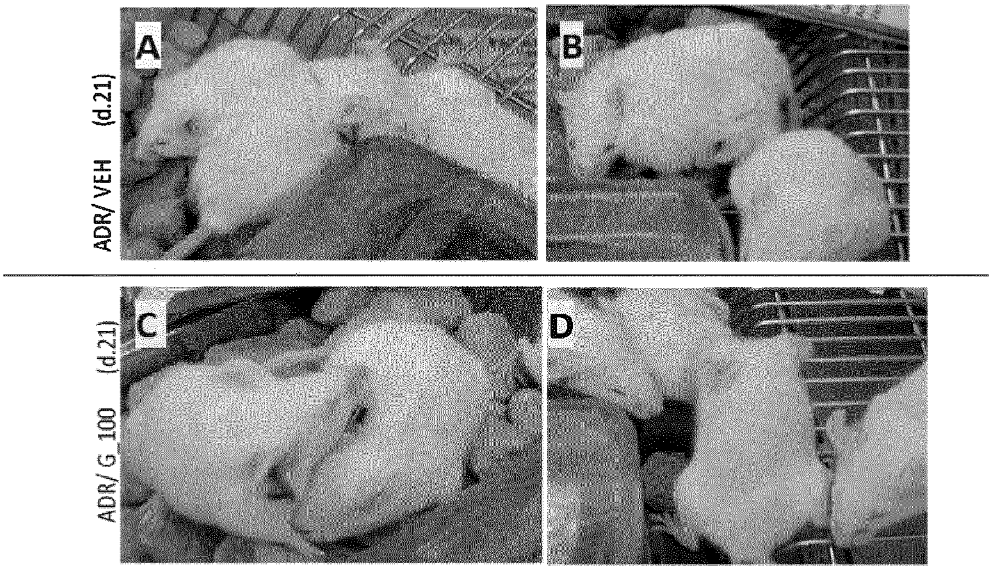


FIGURE 11

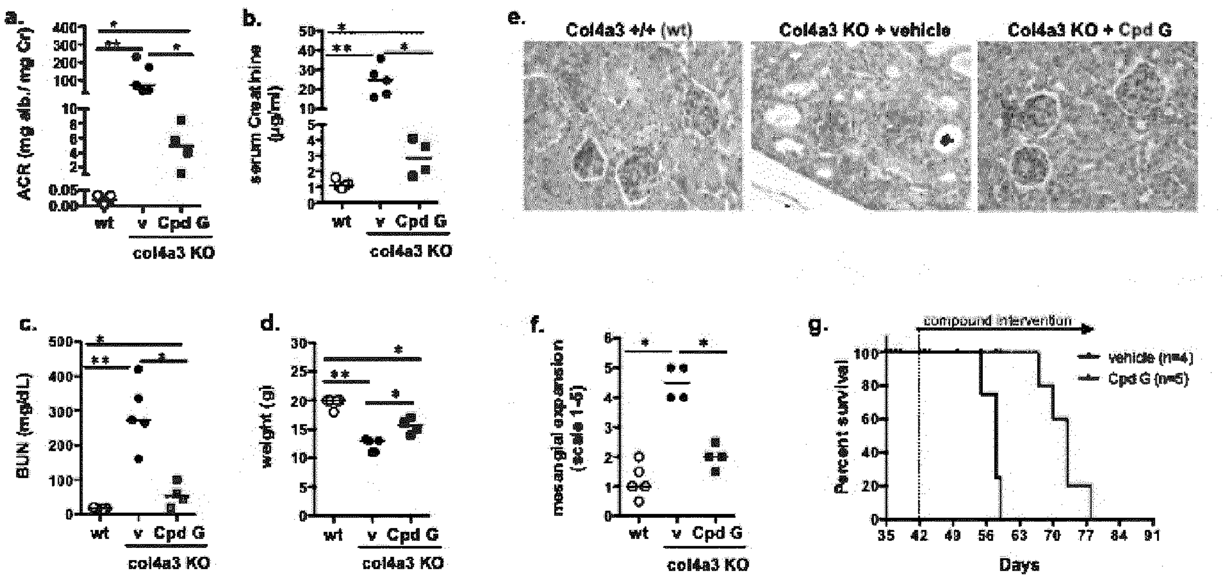

FIGURE 12

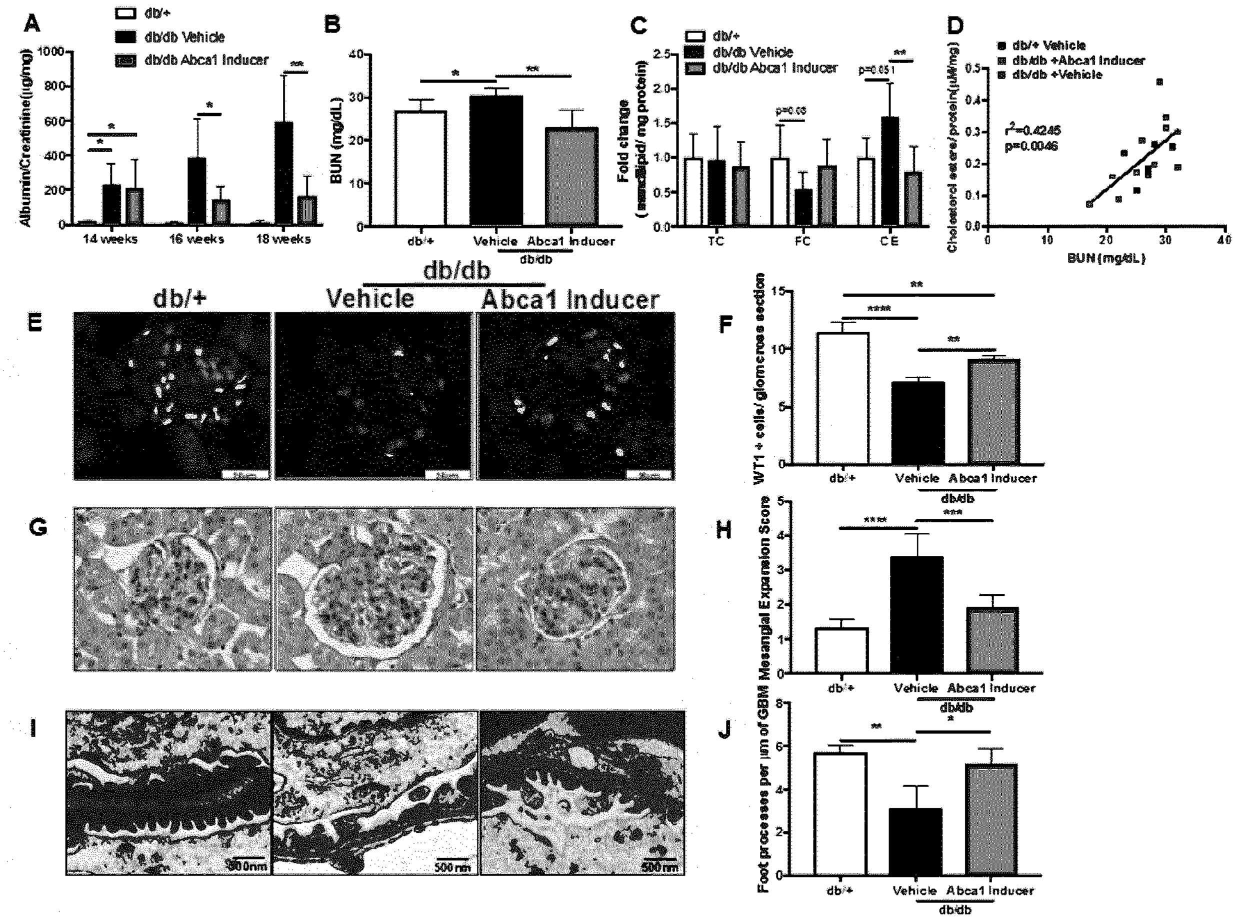


FIGURE 13

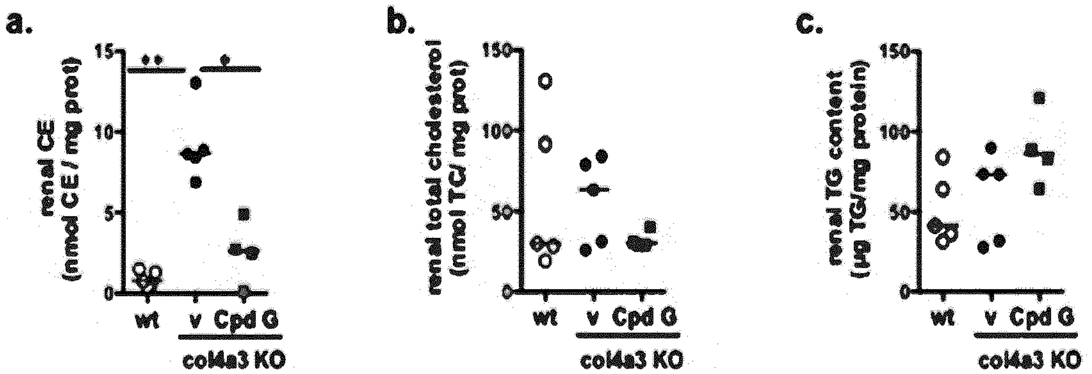

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2011029827 A
- WO 2012032018 A
- WO 2013037703 A
- WO 2014180741 A
- WO 2012031817 A
- WO 201337703 A
- US 5496807 A
- US 7030146 B
- WO 2005105791 A
- WO 2005092856 A

### Non-patent literature cited in the description

- **BELLO AK et al.** *JAMA*, 2017, vol. 317, 1864
- **WILLIAMS** ; **TUTTLE**. *Advances in Chronic Kidney Disease*, 2005, vol. 12 (2), 212-222
- **GIUNTI et al.** *Minerva Medica*, 2006, vol. 97, 241-62
- *CHEMICAL ABSTRACTS*, 52208-50-1
- *CHEMICAL ABSTRACTS*, 95464-05-4
- *CHEMICAL ABSTRACTS*, 125700-67-6
- *CHEMICAL ABSTRACTS*, 13374-31-7
- *CHEMICAL ABSTRACTS*, 1211589-51-3
- *CHEMICAL ABSTRACTS*, 850623-36-8
- *CHEMICAL ABSTRACTS*, 321921-71-5
- *CHEMICAL ABSTRACTS*, 543713-30-0